Europäisches Patentamt

European Patent Office

Office européen des brevets

(11) Publication number: **0 164 176 B1**

## EUROPEAN PATENT SPECIFICATION

(45) Date of publication of patent specification: **09.09.92**

(51) Int. Cl.⁵: **A61K 39/012**, C12N 15/00, C07K 13/00

(21) Application number: **85200889.5**

(22) Date of filing: **05.06.85**

(54) Antigenic proteins and vaccines containing them and protective antibodies directed to them for prevention of coccidiosis caused by Eimeria tenella.

(30) Priority: **05.06.84 US 617483**
**16.05.85 US 734085**

(43) Date of publication of application:
**11.12.85 Bulletin 85/50**

(45) Publication of the grant of the patent:
**09.09.92 Bulletin 92/37**

(84) Designated Contracting States:
**AT BE CH DE FR GB IT LI LU NL SE**

(56) References cited:
**EP-A- 0 135 712**
**EP-A- 0 167 443**

**J. CELL BIOCHEM., Suppl. 1983, vol. 0, no. 7, Part A, 12th Annual Symp. on Melecular Biology of Host-Parasite Interactions, January 30 - February 4, 1983, page 25, ref.no. 0059, M.H. WISHER: "Sporozoite antigens of coccidia."**

(73) Proprietor: **SOLVAY**
**Rue du Prince Albert, 33**
**B-1050 Bruxelles(BE)**

(72) Inventor: **Newman, Jr., Karel Z.**
**Kelly Street 306**
**Charles City Iowa 50616(US)**
Inventor: **Tedesco, John L.**
**311 Danbury Drive**
**Charles City Iowa 50616(US)**
Inventor: **Simonson, Randy R.**
**RR No. 1**
**Charles City Iowa 50616(US)**
Inventor: **Gore, Thomas C.**
**1106 Hildreth Street**
**Charles City Iowa 50616(US)**
Inventor: **Petersen, Gary R.**
**210 2nd Avenue**
**Charles City Iowa 50616(US)**
Inventor: **Brothers, Virginia Mary**
**988 Peralta Avenue**
**Albany California 94706(US)**

EP 0 164 176 B1

PROTOZOOL, vol. 30, no. 3, August 1983, pages 548-554; Soc. of Protozoologists, US, C.A. SPEER et al.: "Ultrastructural localization of monoclonal IgG antibodies for antigenic sites of Eimeria tenella oocysts, sporocysts, and sporozoites."

JOURNAL OF PARASITOLOGY, vol. 68, no. 3, June 1982, pages 392-397; Am. Soc. of Parasitologists, US H.D. DANFORTH: "Development of hybridoma-produced antibodies directed against Eimeria tenella and E. mitis."

Inventor: **Files, James Gordon**
**1911, Lyon Avenue**
**Belmont California 94002(US)**
Inventor: **Paul, Leland Shawn**
**14826 Skyline**
**Woodside California 94062(US)**

(74) Representative: **Smulders, Theodorus A.H.J., Ir. et al**
**Vereenigde Octrooibureaux Nieuwe Parklaan 97**
**NL-2587 BN 's-Gravenhage(NL)**

**Description**

This application is a continuation-in-part of U.S. Serial No. 617,483, filed June 5, 1984, the contents of which are hereby incorporated by reference into the present application.

Throughout this application various publications are referenced by arabic numerals within parentheses. Full citations for these references may be found at the end of the specification immediately preceding the claims. The disclosures of these publications in their entireties are hereby incorporated by reference into this application in order to more fully describe the state of the art as known to those skilled therein as of the date of the invention described and claimed herein.

The phylum Apicomplexa includes hundreds of different organisms belonging to the order Eucoccidiorida. The genus Eimeria is included within the order of true coccidian agents. Of the organisms belonging to this genus, several species are of recognized importance to the chicken industry. These species include Eimeria tenella, E. maxima, E. acervulina, E. necatrix, E. brunetti, E. mivati, E. mitis and E. praecox.

Differentiation of species is based on the site of infection within the host and oocyst morphology. To date, biochemical markers have not been used for speciation, although differences have been noted for each of the above species.

For Eimeria, the entire life cycle is completed within a single host. The actual stages of the life cycle vary in complexity depending upon the Eimeria species involved. E. tenella has a complex life cycle pattern. Upon being ingested in contaminated feces, food or water, sporulated oocysts excyst within the digestive tract as a result of the combined action of mechanical shearing and enzymatic hydrolysis of the sporocyst cap. The liberated sporozoites traverse epithelial cells within specific regions of the cecum. Development begins within the Crypt of Lieberkühn to the level of first generation meronts; the meront is a transitional stage consisting of rounded organisms with a more pronounced nucleus, plus increased energy generating and protein synthesizing capacity. Development of first-generation merozoites follows due to multiple fission of meronts. The release of first-generation merozoites destroys the host cell, and the parasites migrate to infect new host cells undergoing a second asexual cycle. Meronts develop to the level of second-generation merozoites destroying additional epithelial cells as they are released. Further destruction of host cells follows with the liberation of the third-generation merozoites. Second- and third-generation merozoites may infest still another population of host enterocytes to begin the sexual phase. Sexual development commences with the production of microgametes and macrogametes through the process of gametogenesis. Liberated microgametes fertilize macrogametes to form zygotes. Development of immature oocysts is followed by rupture of the host cell. Oocysts, released into the lumen of the gut, are passed through the feces to the environment and mature (sporulate) in the presence of atmospheric oxygen.

The process of parasite development is self-limiting if the host ingests no additional oocysts. However, this proves to be an unrealistic expectation in crowded poultry houses. Disease due to E. tenella results in severe losses because of pathologic manifestations.

The pathology of coccidiosis due to E. tenella and some other species is in large part related to the rupture of host cells during the release of merozoites. Tissues are disrupted primarily within the gut lamina propria; in theory, ingestion of a single oocyst can result in the destruction of up to $2.5 \times 10^6$ host cells within 120 hours (45). Bleeding within the gut is related to rupture of small capillaries servicing the epithelium. It is difficult to control the progress of disease using coccidiostats, once established. Secondary infection often complicates the disease caused by Eimeria. Death generally occurs within 4-7 days in infected birds.

A consistent property of the coccidia is that the sporozoites initiate the infection process within very specific tissue sites (28, 23, 41). The site specificity of infection is a characteristic commonly used for speciation of Eimeria. E. tenella shows a propensity for invasion of epithelial cells residing within the posterior portion of the cecum (cecal pouches). In part, such specificity for infection may depend upon the interaction of parasite surface determinants with the host cell type, or specific surface components (9, 11, 40). This contention is also supported by studies with genetically "resistant" birds, the cells of which are not invaded by Eimeria within the normal site of colonization. Work with other coccidia clearly demonstrates that the expression of requisite host determinant(s) is directed by the tissue origin of cells in addition to the host cell mitotic state (11).

Much of the work on immunity to coccidiosis has been confined to humoral immunity, and more specifically to serum antibody. The studies have shown a lack of correlation between serum antibody and resistance to disease (43). However, most available data suggest that a local response with involvement of the secretory immune system or cell mediated immunity (CMI), or both, are involved in the protective response.

3

Interference with recognition and/or attachment of pathogens to host cells has a demonstrated protective effect as shown with viral, bacterial and protozoan agents. Genetic deletion of key host cell receptors or pathogen attachment features can prevent the initial colonization process (14, 40). Alternatively, secretory antibodies can interfere with the colonization process by binding to, and consequently masking requisite host cell recognition determinants (22, 54). All immunoglobulin classes tested to date have been reported to have the capacity of interfering with the initial colonization process of Eimeria tenella (10). However, recent reports indicate that only production of secretory IgA has been correlated with natural protective immunity (9, 43). Porter and Davis (10) and others (43) reported that secretory IgA neutralizes the extracellular stages of the parasite by significantly limiting penetration or so debilitating those organisms which did penetrate as to prevent subsequent development.

It has been estimated that an amount approaching $0.5-1.0 billion is spent annually by producers worldwide in an effort to curb the devastating effect of coccidiosis in chickens (28, 39). Even with control measures currently in use, poultry losses are substantial with estimates in the multi-million dollar range (45).

Currently, the most widely used means of controlling Eimeria in chickens is through the application of antiprotozoal chemical feed additives. The specific composition varies with the coccidiostat used, and each product affects only certain stages of the coccidian life cycle (28, 38, 42). Disadvantages of using coccidiostats are many, including short-term residual protection in birds, occasional diminished performance, invocation of resistance to the drug in parasites, and to some extent, safety. Products currently remain on the market for only a few years because of the development of drug resistant strains. This adds considerable pressure on the cost of development and continued manufacture of efficacious products (38).

Protection of birds by immunization has met with some success. Investigators have been able to invoke limited protection using preparations of killed organisms (1, 30, 31). A more effective approach for immunization of chickens has been with the use of a live protozoal product-e.g. Coccivac® (13). The product, being a multivalent composition containing low doses of viable oocysts, is administered in drinking water to invoke a mild parasitemia in birds. A drawback of this product has been occasional depressed performance of birds during the first weeks following administration. Variables such as excessive dosing or moisture content of bedding have even led to severe outbreaks of coccidiosis. See also, U.S. Patent No. 3,147,186 (1964) which concerns the use of viable, sporulated oocysts of E. tenella to immunize chickens and U.S. Patent No. 4,301,148 (1981) which concerns the use of sporozoites of E. tenella for the same purpose.

An alternative means of introducing the live vaccine into broiler houses is by way of the feed. This has been considered in a recent British patent (GB2,008,404A). Prior to mixing with the feed, fully virulent oocysts of E. tenella are encapsulated in a water soluble polysaccharide to protect against dessication. The oocysts are in sufficient amounts only to induce subclinical infection. Though the immunizing ability was found to be excellent, no development of this method is foreseen due to questionable field acceptability. However, if attentuated strains of all the important coccidia could be developed, the procedure may be more acceptable.

Efforts have indeed been made to develop Eimeria lines of reduced virulence. Some species have been successfully attenuated through chicken embryo passage (15, 26, 29, 48). These strains have diminished ability to cause disease, yet have retained sufficient immunogenicity to invoke immunity. Some problems do, however, remain with the handling of these strains. As examples, the attenuated variants of E. necatrix have a critical passage limit whereby more or less embryo passage can result in loss of immunogenicity or maintenance of the original virulent form. Furthermore, some attenuated organisms revert to the virulent form upon minimal back-passage through chickens (27, 50). Thus, problems associated with maintaining consistent properties in attenuated organisms are apparent.

Attenuation by precocious selection has also been practiced when Eimeria strains cannot be readily passaged through embryonated eggs. In this process, shed oocysts are harvested late in the prepatent period prior to the onset of heavy oocyst shedding (18, 35, 37, 49). Such selection results in cultures having abbreviated life cycles, and a corresponding diminution in virulence properties (18, 35, 37, 49). Though the trait of precocity for E. tenella (19) and E. acervulina (36) has been demonstrated to be genetically stable, not enough information is known about this method to assess its usefulness as a tool in the poultry industry. There is little information available about the surface antigen composition of avian coccidia. Hybridoma cell lines which secrete monoclonal antibodies directed to antigens on the surface of sporozoites of Eimeria tenella have been reported (58). The antigens were not identified, other than that their molecular weights were between 13 and 150 kilodaltons. Moreover, no biological significance or described efficacy in a vaccine was attributed to the antigens. Previous work in the laboratory of M.H. Wisher suggests the presence of approximately 16 polypeptides identified by surface iodination of excysted sporozoites of E. tenella and having molecular weights from 20,000 to greater than 200,000 (57).

4

Subunit approaches to vaccine development have proven successful over the past few years. In such approaches, candidate protective antigens are identified and characterized for purpose of eventual preparation on a large scale. In studying parasite antigens, one research group used monoclonal antibodies to identify a potential protective antigen on the surface of Babesia bovis (59). A B. bovis antigen of 44,000 daltons has been identified, which when purified and injected into experimental animals afforded some level of protection against primary challenge. An immunologically important 30,000 dalton protein of Toxoplasma gondii has also been identified using monoclonal antibodies (21).

Since mid-1981, Danforth and coworkers have published several papers in which they indicate the possibility of producing monoclonal antibodies toward antigens of avian Eimeria species (6, 7, 8). Similarly, Speer, et al (51, 52) have demonstrated the development of hybridomas against E. tenella and some physiologic properties thereof. Antibody-secreting hybridomas were selected on the basis of an indirect fluorescent antibody test (7). The patterns of reaction, as observed with ultraviolet microscopy, have varied depending upon the monoclonal antibody used. Patterns have included exclusive reaction with sporozoites only vs reaction with sporozoites and merozoites; staining of the anterior portion of the sporozoite vs the entire membrane; and staining of distinct internal organelles vs non-descript internal staining (8).

Although the preparation of murine-origin hybridomas producing monoclonal antibodies is known, there is nothing to suggest that the direct and specific selection of sporozoite-neutralizing hybridomas will identify virulence determinants of E. tenella useful in the development of a subunit vaccine.

In fact, there is no prior teaching concerning identification of antigens of Eimeria useful for subunit vaccines. Similarly, there has been no prior teaching of the formulation and use of products which include monoclonal antibodies to confer passive immunity against E. tenella.

This invention concerns the identification, characterization, preparation and use of polypeptide antigens for development of immunity to coccidiosis by Eimeria tenella. The antigens are capable of being precisely dispensed in terms of direct antigenic content and cannot cause disease thus avoiding vaccine strain-related outbreaks and reversions or changes in immunologic properties.

The invention also concerns the preparation, formulation and use of a monoclonal antibody product directed against the aforementioned protective protein antigen which can be used in the purification of the antigen, as well as to confer passive protection against coccidiosis due to E. tenella in chickens.

## Summary of the Invention

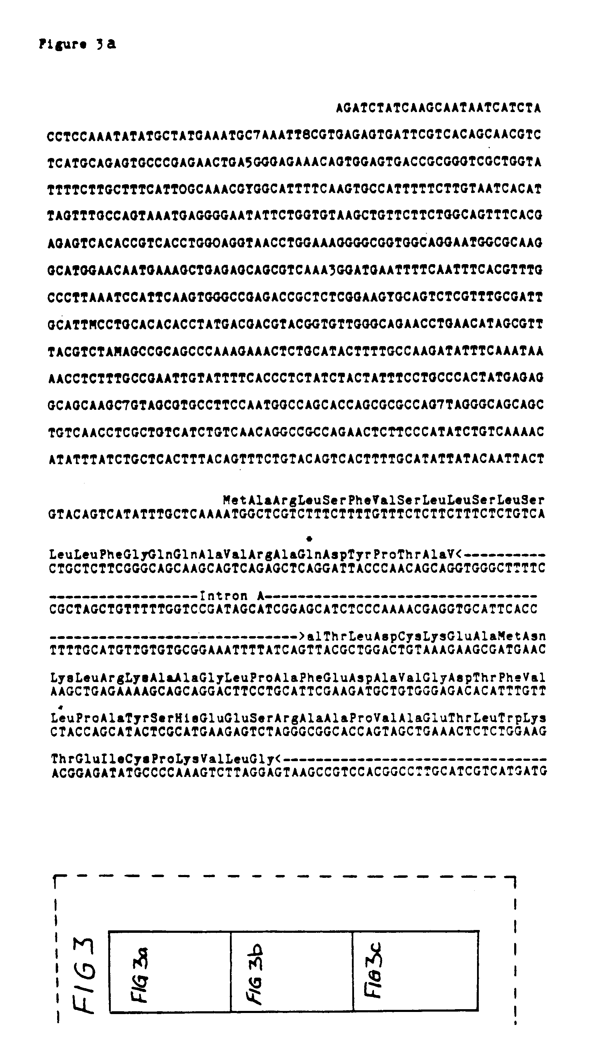

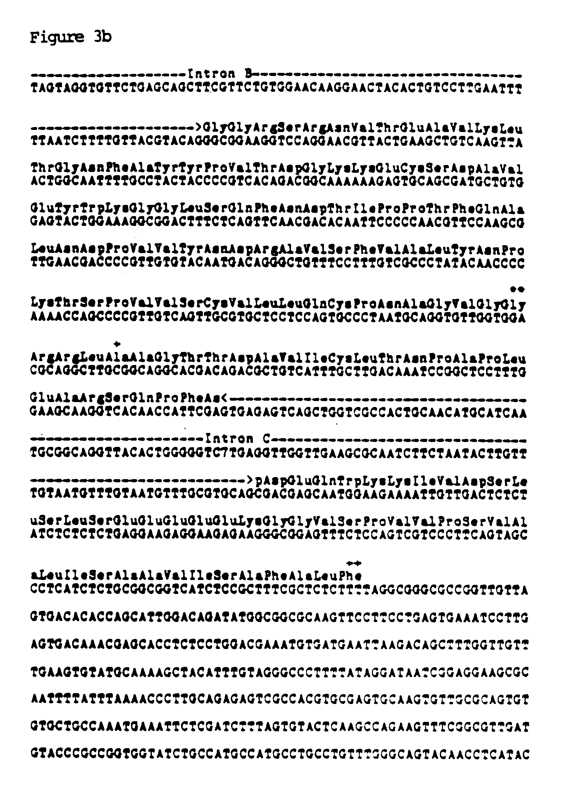

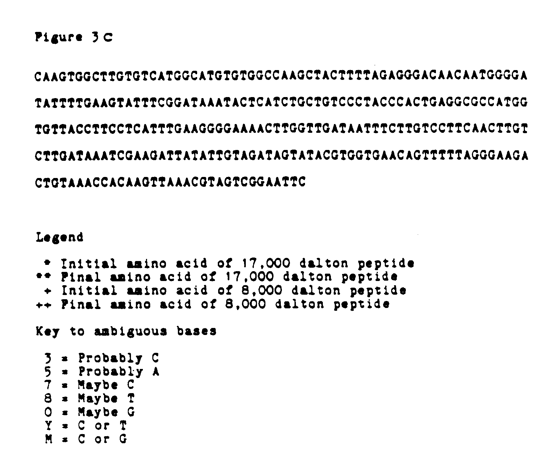

A purified antigenic protein, also referred to herein as the A4 protein or A4 antigen, has been obtained which is capable of inducing in a chicken an immune response conferring protection against infection by Eimeria tenella. The protein has a molecular weight of about 25,000 and is composed of two polypeptides joined by a disulfide bond. One of the polypeptides is characterized by a molecular weight of about 17,000 and by a blocked N-terminal amino acid. The other of the polypeptides is characterized by a molecular weight of about 8,000. The amino acid sequences of both polypeptides are set forth in Figure 3.

Polypeptides related to the A4 protein may be obtained which are characterized by the ability to induce in a chicken an immune response conferring protection against infection by E. tenella. Two such polypeptides have been prepared having apparent molecular weights of about 11,500 and 6,500, respectively, when determined by SDS-polyacrylamide gel electrophoresis under reducing conditions.

The 25,000 molecular weight, purified protein antigen may be prepared by separately recovering it from the sporocyst of Eimeria tenella. In one preferred embodiment recovery involves immunoadsorption chromatography or immunoprecipitation utilizing the highly specific monoclonal antibody Ptn 7.2A4/4 produced by hybridoma cell line ATCC No. HB8561. Alternatively, the protein may be prepared by recombinant DNA technology utilizing a DNA molecule encoding the protein. The nucleotide sequence of such a DNA molecule is depicted in Figure 3. The 11,500 and 6,500 dalton polypeptides may be similarly prepared.

Active immunity against infection by E. tenella may be conferred upon a chicken by administering to the chicken an effective-immunizing amount of the 25,000 dalton protein antigen or of an antigenic polypeptide of this invention, e.g. the 11,500 and 6,500 dalton polypeptides. Preferably, the protein or polypeptide is incorporated into a vaccine with a suitable carrier and suitable doses of the vaccine administered to the chicken.

The monoclonal antibody Ptn 7.2A4/4 or any other such antibody may be used to confer passive immunity against E. tenella infection, preferably in admixture with a suitable carrier. In addition, anti-idiotype antibody directed against the monoclonal antibody may be produced and used to confer active immunity against infection by E. tenella. Preferably, the anti-idiotype antibody is administered with a suitable carrier in the form of a vaccine.

Brief Description of the Figures

Figure 1 displays the amino acid sequence of the 17,000 dalton polypeptide component of the E. tenella A4 antigen determined by microsequencing. Figure 1 also shows the overlapping peptides produced by various chemical and enzymatic digestions.

Figure 2 shows the restriction enzyme maps of the E. tenella genomic clone encoding the A4 antigen. Figure 2 also shows the position and orientation of the gene for the A4 antigen within the 5500 bp E. tenella Eco RI DNA fragment.

Figure 3 shows the DNA nucleotide sequence of the Bgl II-Eco RI DNA fragment of the genomic clone depicted in Figure 2. In addition, Figure 3 shows the amino acid sequence for the signal peptide and the 17,000 dalton and the 8,000 dalton polypeptide components of the A4 antigen. Figure 3 also shows the introns within the gene.

Detailed Description of the Invention

This invention concerns a purified antigenic protein capable of inducing in a chicken an immune response conferring protection against infection by Eimeria tenella. The protein has a molecular weight of about 25,000 and is composed of two polypeptides joined by a disulfide bond. One of the polypeptides is characterized by a molecular weight of about 17,000 and by a blocked N-terminal amino acid and has the amino acid sequence set forth in Figure 3. The other of the polypeptides is characterized by a molecular weight of about 8,000 and has the amino acid sequence set forth in Figure 3.

The 25,000 dalton protein, also referred to herein as the A4 protein or A4 antigen has been obtained from sporocysts of Eimeria tenella. Although the protein has been derived from E. tenella, it is contemplated that the protein may be prepared by other methods, e.g., recombinant DNA technology or total organic synthesis and accordingly the invention is not limited to protein prepared directly from E. tenella but encompasses the protein per se independent of its method of preparation.

If the A4 antigen is radiolabeled by iodination of E. tenella sporocyst membrane preparations, the iodinated protein has an apparent molecular weight of 17,000 as determined by SDS-polyacrylamide gel electrophoresis (SDS-PAGE) (24) under reducing conditions and detected by autoradiography. Under such reducing conditions the 8,000 dalton polypeptide is separated from the 17,000 dalton polypeptide and is not detected by autoradiography. The isoelectric point of the A4 protein is about 5.2 as determined by the method of Wrigley (45). The N-terminal amino acid of the 17,000 dalton component of the protein is blocked. Before its complete amino acid sequence was known, the protein was initially characterized by microsequencing analysis and found to contain within it the amino acid sequences:

```
1                    5
Ala  Val  Lys  Leu  Thr  Gly  Asn  Phe  Ala  Tyr

11                   15                        20
Tyr  Pro  Val  Thr  Asp  Gly  Lys  Lys  Glu  Cys

21                   25                        30
Ser  Asp  Ala  Val  Glu  Tyr  Trp  Lys  Gly  Gly

31                   35
Leu  Ser  Gln  Phe  Asn

                     and

1                    5                         10
Thr  Leu  Trp  Lys  Thr  Glu  Ile  Cys  Pro  Lys

11                   15                        20
Val  Leu  Gly  Gly  Gly  Arg  Ser  Arg  Asn  Val

21
Thr  Glu
```

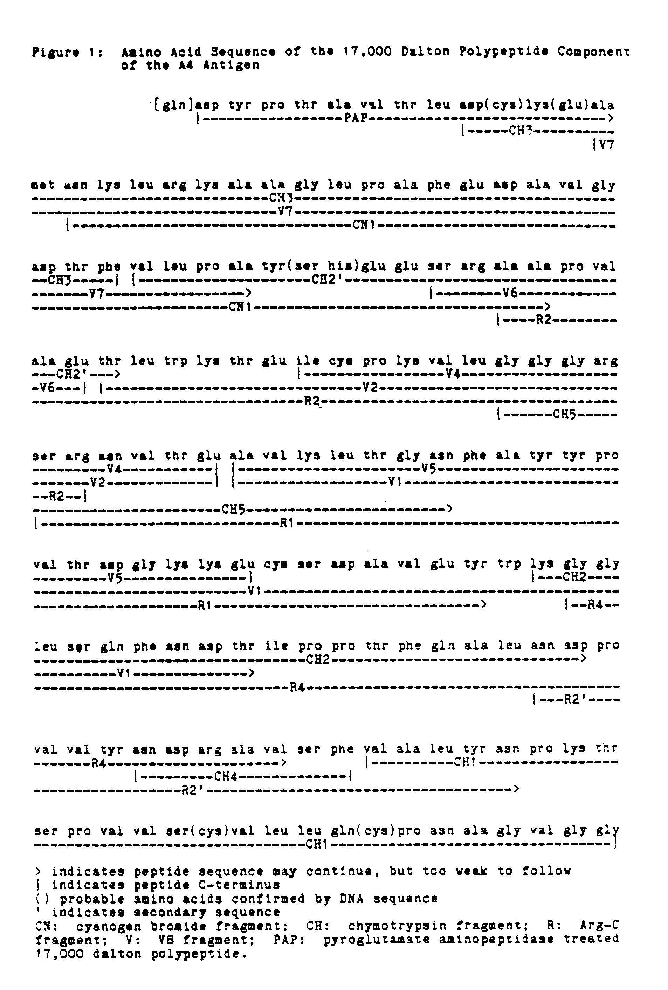

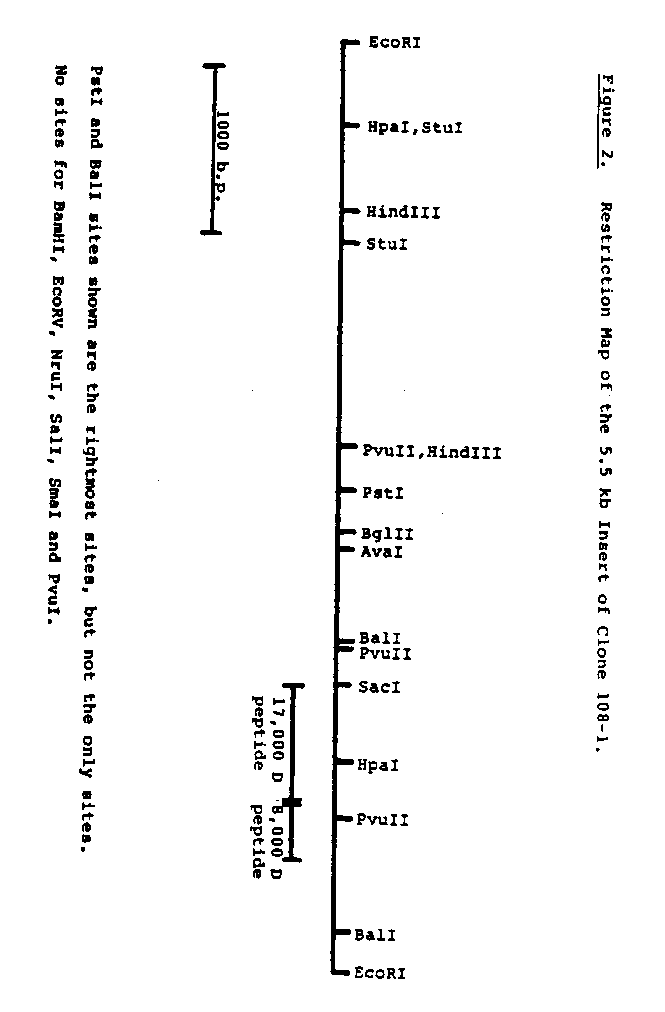

The presence of these amino acid sequences was confirmed by elucidation of the complete amino acid sequence (Figure 1, 3). Blockage of the N-terminus of the protein is also supported by the complete amino acid sequence which indicates the presence of an N-terminal glutamine molecule. Such glutamine residues are known to cyclize to form pyrrolidone carboxylic acid.

The A4 protein has an actual molecular weight of about 25,000 based on its complete amino acid sequence, and has an apparent molecular weight of 21,000-23,000 on SDS-PAGE under non-reducing conditions. After reduction with β-mercaptoethanoltwo polypeptides are seen on SDS-PAGE (24). The A4 protein therefore consists of two polypeptides, a 17,000 and an 8,000 dalton peptide which are bonded to one another by disulfide linkage. The complete amino acid sequence of the 17,000 dalton peptide and a partial amino acid sequence of the 8,000 dalton peptide have been determined by microsequencing analysis. The sequences so determined are consistent with the separately determined sequence of the chromosomal DNA which encodes the protein.

This invention also contemplates antigenic polypeptides which include an amino acid sequence present within the A4 protein and which are capable of inducing in a chicken an immune response conferring protection against infection by Eimeria tenella. Such polypeptides include all amino acid sequences which contain an antigenic determinant from the A4 protein and which are capable of inducing an immune response.

Two preparations containing two such polypeptides have been prepared. These preparations are characterized by the presence within them of polypeptides with apparent molecular weights of about 11,500 and about 6,500 respectively, on SDS-polyacrylamide gel electrophoresis under reducing conditions. These preparations have been identified immunologically as derived from the A4 antigen.

Antigenic polypeptides which include an amino acid sequence present in the A4 protein may be produced by various methods, e.g., may be chemically or enzymatically synthesized, may be produced by recombinant DNA methods, may be prepared from the A4 antigen or may be prepared from the sporocyst or sporozoite of E. tenella. It should be understood that "antigenic polypeptide" as the term is used herein includes preparations prepared under non-reducing conditions as described herein, characterized by the presence within the preparation of a polypeptide having a defined apparent molecular weight on SDS-PAGE under reducing conditions. When present in such preparation, the polypeptide may be bound to another component or components, e.g. to another polypeptide by one or more disulfide bonds or two or more regions within the polypeptide may be bound to one another, e.g. by a disulfide bond. For those preparations characterized by the presence within them of polypeptides with apparent molecular weights of 17,000 or less on SDS-PAGE under reducing conditions the term "fragment" is also used to describe such preparations on the assumption that the preparations include amino acid sequences contained within the

7

complete A4 protein, but not the intact protein. In addition the term "fragment" is used to describe amino acid sequences derived from the A4 protein by proteolytic digestion.

The antigens of this invention in addition to an amino acid sequence included within the amino acid sequence of the A4 protein may contain one or more other substances such as polysaccharides, e.g. dextran, or other amino acid sequences, i.e. amino acid sequences not included within the amino acid sequence set forth in Figure 3.

This invention also concerns a method for preparing the A4 protein. This method involves contacting sporocysts of E. tenella with a detergent under suitable non-reducing conditions in the presence of protease inhibitors so as to solubilize the sporocyst membrane proteins. The protein is then separately recovered from the solubilized sporocyst membrane proteins using methods for separating and purifying proteins. Such methods are known to those of ordinary skill in the art to which this invention pertains and include by way of example, partial purification of the solubilized, sporocyst membranes by ion exchange chromatography, e.g. on DEAE-cellulose, and hydroxyapatite HPLC.

Alternatively, the A4 protein is separately recovered from an extract of E. tenella sporocyst membrane proteins by immunoprecipitation or immunoadsorption chromatography utilizing a monoclonal antibody directed against the A4 protein such as the monoclonal antibody designated Ptn 7.2A4/4, which antibody is produced by a mouse hybridoma cell line deposited with the American Type Culture Collection in Rockville, Maryland, U.S.A. 20852 under ATCC No. HB8561. This deposit was made pursuant to the provisions of the Budapest Treaty on the International Recognition of the Deposit of Microorganisms.

The 17,000 dalton polypeptide of the A4 protein may be prepared by the same method used in preparing the A4 protein. The 17,000 dalton poypeptide is then separately recovered, e.g. by effecting partial purification with ion-exchange chromatography, e.g. with DEAE-cellulose, followed by preparative SDS-electrophoresis under reducing conditions.

The 11,500 dalton antigenic polypeptide may be prepared by contacting sporocysts of E. tenella with phenol in the presence of a mild reducing agent, e.g. 8-hydroxyquinoline, so as to extract the sporocyst membrane proteins. The polypeptide is then recovered from the extracted sporocyst membrane proteins by immunoprecipitation or immunoaffinity chromatography with an appropriate antibody such as the Ptn 7.2A4/4 monoclonal antibody. The 6,500 dalton antigenic polypeptide may be prepared by contacting membranes of trypsin-taurodeoxycholate excysted sporozoites ( 9) with detergent so as to solubilize the proteins. The polypeptide is then recovered from the solubilized, excysted proteins by immunoprecipitation or immunoadsorption chromatography, again, with an appropriate antibody such as the monoclonal antibody Ptn 7.2A4/4.

A method involving recombinant DNA technology is also provided for preparing the 25,000 dalton A4 protein, the 17,000 dalton polypeptide thereof or the various antigenic polypeptides of this invention. The method involves preparing a DNA molecule coding for the A4 protein or polypeptide and inserting the DNA molecule into an appropriate expression vector, e.g. a vector containing the $\lambda P_L$ or lac promoter. The resulting expression vector is then introduced into a suitable host, e.g. E. coli, under appropriate conditions permitting expression of the DNA and production of the protein or polypeptide, which is then recovered.

Messenger RNA may be isolated from sporocysts at many points during the sporulation process. These mRNA samples may then be translated using in vitro (32) or in vivo systems. The translation products may then be immunoprecipitated using the monoclonal antibody (Ptn 7.2A4/4) or anti-sporozoite chicken serum. The mRNA preparation encoding the A4 antigen may then be used to produce double-stranded cDNA (32). This cDNA may then be inserted into an appropriate cloning vector which may then be used to transform E. coli to generate a cDNA library. This cDNA library may then be screened by colony hybridization techniques using isotopically-labelled oligonucleotide probes whose construction is based upon amino acid sequence information from the 17,000 dalton polypeptide component of the A4 antigen. Vector DNA from bacterial colonies containing nucleotide sequences for the 17,000 dalton polypeptide may then be isolated and the inserted coccidial DNA sequenced (34, 44).

Alternatively, genomic DNA from E. tenella has been isolated and cleaved with a restriction endonuclease such as EcoRI. The restriction fragments were ligated into an appropriate cloning vector such as λgt wes λB generating a genomic library. The genomic library was then be screened by plaque hybridization as was described for the cDNA library. A genomic clone encoding the A4 antigen has been isolated and the DNA sequence has shown the two peptides of the A4 antigen to be encoded by a contiguous nucleotide sequence (Figure 3). Hence the 17,000 and 8,000 dalton peptides are derived from proteolytic processing of a single 25,000 dalton peptide. In addition the DNA sequence encodes a "signal" sequence typically found at the amino terminus of many secretory or membrane proteins.

This invention also encompasses a method for conferring upon a chicken active immunity against infection by Eimeria tenella which comprises administering to a chicken an effective immunizing amount of the A4 antigen, an antigenic polypeptide or other antigen of this invention, including but not limited to the 11,500 dalton or the 6,500 dalton polypeptides. By this method active immunity may be conferred upon a non-immune chicken. In addition, administration of these materials may be used to increase a relatively low level of immunity in a chicken previously exposed to E. tenella and may be used in booster vaccinations.

The A4 antigen or any of the antigenic polypeptides of this invention may be administered to chickens by any of a number of well known methods. Desirably, the administration may involve subcutaneous or intramuscular injection at the back of the neck. The amount of antigen comprising an effective immunizing amount may be any amount from about 0.1 microgram to about 1 mg. The amount of antigen is desirably above about 10 micrograms. The preferred amount of antigen is about 500 micrograms per kilogram of body weight. Alternatively, the administration may be oral (e.g., via capsule) or desirably by injection (e.g., subcutaneous, intradermal, or preferably intramuscular injection). If the mode of administration involves injection, any pharmaceutically acceptable carrier may be employed. Suitable carriers include 0.01 to 0.1M, preferably 0.05M, phosphate buffer or 0.8 percent saline.

A vaccine for conferring upon a chicken active immunity against infection by Eimeria tenella is provided which comprises an effective immunizing amount of an antigenic material of this invention, i.e. the A4 antigen, antigenic polypeptide or other antigen of this invention, and a suitable carrier. Preferably the effective immunizing amount of the antigenic material in the vaccine is above about 0.1 microgram/kg of body weight of the chicken.

In addition, the carrier desirably also contains a preservative. One particularly suitable preservative is thimerosal (sodium ethylmercurithiosalicylate) which has activity as both a bacteriostat and a fungistat. Desirably thimerosal is present in the vaccine in a final concentration of $10^{-4}$ percent.

Furthermore, the carrier desirably also contains an immunopotentiator. Various immunopotentiators known in the art may be used. The adjuvant presently employed is 94% Drakeol 6-VR, 5% Arlacel A, 1% Tween-80. Arlacel A is a mannide monoleate (Sandria Corp.). It is an irritant which has strong immunopotentiating activity when combined with antigens. Drakeol 6-VR is a hypoallergenic light mineral oil product (Penreco Corp.). Tween-80 is a monoleate derivative of polyoxyethylsorbitan and possesses detergent properties. Other suitable carriers or immunopotentiators include aluminum potassium sulfate, aluminum hydroxide, lymphokines and water in oil emulsions.

By administering a suitable dose of such a vaccine to a chicken, the chicken is protected against infection by E. tenella. The amount of antigenic material per dose should be sufficient to induce production of antibodies to the antigenic material in an animal to which the vaccine is administered. To provide a sufficient degree of immunological response as measured by antibody production and protection, the amount of the antigenic material per dose is desirably above about 20.0 micrograms/kg of body weight of the vaccinated animal. Thus, the amount of antigenic material based upon a 50 gram day-old chick would be above about 1.0 microgram. Presently preferred is a vaccine containing 10 micrograms of antigenic material. In general, the antigen will comprise on a weight basis from about 0.002 percent up to about 0.2 percent of the vaccine and the dose volume will be about 0.1 ml.

Other embodiments of this invention include a monoclonal antibody directed against the A4 protein and a monoclonal antibody directed against an antigenic polypeptide of this invention. A specific embodiment is the previously mentioned monoclonal antibody designated Ptn 7.2A4/4 produced by hybridoma cell line ATCC No. HB8561.

One can confer upon a chicken passive immunity against infection by E. tenella by administering to a chicken an effective protecting amount of a monoclonal antibody directed against the A4 antigen or antigenic fragment thereof, e.g. the monoclonal antibody Ptn 7.2A4/4. A composition useful to this end comprises an effective protecting amount of an appropriate monoclonal antibody, e.g. monoclonal antibody Ptn 7.2A4/4, and a suitable carrier. Said composition may consist of a sufficient dose of the monoclonal antibody so as to protect against infection when administered via the oral route. A typical dose of antibody can be about 100 micrograms of antibody per bird per day administered in either aqueous or lyophilized form. Preferably, the composition is used in aqueous form as an addition to the water supply. The antibody is dissolved in 0.15M phosphate buffered saline, pH 7, containing 0.0001 percent thimerosal to a final protein content of 1-100 mg/ml. The product is continuously dispensed within the water so as to maintain the desired antibody level. Administering to a chicken a suitable dose of such a composition is thus a method for conferring passive immunity against infection by E. tenella.

In a further embodiment a composite structure having spatial features common with those of the principal protective structures of the A4 antigen is substituted for the previously described antigens. One such composition includes an anti-idiotypic antibody developed against the structures of an antibody to the A4 protein or to one of the antigenic polypeptides of this invention, e.g. the monoclonal antibody Ptn 7.2A4/4, which structures confer specificity toward the respective antigen determinant. Such anti-idiotype antibodies can in themselves be monoclonal in nature, or can be raised as polyclonal antibodies. In the former example, the antibody Ptn 7.2A4/4 can be recovered from hybridoma cell line ATCC No. HB8561, purified and covalently attached to any suitable carrier protein, e.g. the key-hole limpet hemocyanin (KLH). The purified antibody, preferably the purified antibody-KLH complex, is repeatedly injected, preferably with an adjuvant such as Freund's complete adjuvant, into a suitable mammalian lymphocyte donor with Balb/C strain mice as the preferred donor. Hybridomas are developed from lymphocytes of the immunized mice. The hybridomas are screened for antibodies which compete with the A4 antigen for reaction with the monoclonal antibody Ptn 7.2A4/4, but recognize neither the A4 antigen nor murine immunoglobulin other than Ptn 7.2A4/4. Such hybridomas secreting anti-idiotype antibodies toward monoclonal antibody Ptn 7.2A4/4 are further expanded and cloned. Production of anti-idiotype antibody can be performed by cultures of cells in any medium suitable for growth of hybridomas and expression of monoclonal antibodies, or growth of antibody producing hybridomas in host animals, with Balb/C mice as the preferred vehicle.

Anti-idiotype antibody can also be produced by injection of Ptn 7.2A4/4 into animals. One preferred method is to repeatedly inject 500 micrograms of Ptn 7.2A4/4, purified and formulated in a suitable adjuvant, e.g. Complete Freunds Adjuvant, into a suitable animal, e.g. a rabbit. After sufficient injections, blood serum is removed after a suitable period of time from such animals. The anti-idiotypic antibodies are then recovered from the blood serum, e.g. by adsorption against normal mouse serum proteins immobilized on an insoluble support such as Sepharose®. Specificity of the resulting antiserum is confirmed by demonstrating reactivity with monoclonal antibody Ptn 7.2A4/4 but none against normal murine γ3(K).

The anti-idiotype antibodies prepared as above are further purified to the level of IgG fractions. Purified anti-idiotype antibody protein may be administered by any of a number of well-known methods as described for the antigen proper.

Administering an effective amount of the anti-idiotypic antibody of this invention to a chicken provides a method of conferring upon the chicken active immunity against infection by Eimeria tenella. A vaccine for this purpose comprises an effective immunizing amount of the anti-idiotypic antibody and a suitable carrier. Thus, administering a suitable dose of such a vaccine to a chicken provides a method for protecting the chicken against infection by Eimeria tenella.

The amount of anti-idiotype antibody per dose must be sufficient to invoke production of an antibody in an animal to whom the vaccine is administered. For induction of an immunological response as measured by antibody production, the amount of anti-idiotype antibody per dose is above 50 μg/Kg body weight of the vaccinated birds. Thus, the amount of anti-idiotype antibody administered to a 50g day-old chick would be 2.5μg. Presently preferred is a vaccine containing 25 μg of anti-idiotype antibody. In general, the anti-idiotype antibody will comprise on a weight basis from 0.002 percent to up to 0.2 percent of the vaccine, and the dose volume will be 0.2 cc.

Another aspect of the invention is a nucleic acid molecule, e.g. DNA, cDNA, RNA or mRNA, encoding the A4 protein. One embodiment is a DNA molecule having the nucleic acid sequence set forth in Figure 3. Another embodiment is a DNA molecule encoding one of the antigenic polypeptides of this invention.

A further embodiment is a cloning vehicle which comprises a nucleic acid molecule of this invention, e.g. encoding the A4 protein or one of the previously described antigenic polypeptides. The cloning vehicle may be contained in a host cell, e.g. a bacterial host cell.

The protein having the amino acid sequence set forth in Figure 3 may be produced using host cells containing a cloning vehicle which contains the nucleic acid molecule encoding the A4 protein. In accordance with this method, the host cells are grown under suitable conditions permitting production of the protein, and the protein so produced is recovered. The antigenic polypeptides of this invention may be similarly prepared using an appropriate nucleic acid molecule encoding the polypeptide.

When the host cell is a bacterial cell, the A4 protein produced has the same or essentially the same sequence as the natural A4 protein but may differ in its amino acid sequence or at its amino terminus because of expression in the bacterial host, e.g. by addition of an N-terminal methionine molecule or by virtue of being produced and recovered as a single, unprocessed protein.

A further embodiment concerns a method for obtaining the DNA molecule having the nucleic acid sequence set forth in Figure 3. The method involves isolating genomic DNA from Eimeria tenella oocysts and preparing DNA fragments from the genomic DNA so isolated, e.g. with a restriction enzyme. The DNA fragments are ligated into an appropriate cloning vector. Appropriate clones are identified by subjecting their DNA to hybridization with oligonucleotides containing, or complementary to, nucleic acid sequences present within the nucleic acid sequence set forth in Figure 3. DNA which encodes the protein and has the nucleic acid sequence set forth in Figure 3 is isolated from the appropriate clones.

EXAMPLE 1

## PREPARATION OF EIMERIA TENELLA OOCYSTS, SPOROCYSTS AND SPOROZOITES

Coccidia. The purified field isolate of Eimeria tenella was originally purchased from Dr. Allen Edgar of the University of Auburn. The purity of this E. tenella isolate was confirmed using oocyst characteristics and histology of infected intestinal tissue. Oocyst size and shape index were within the range of E. tenella.

Lesions were scored by the method of Johnson and Reid (20). The lesions in infected birds were typical of E. tenella. The pathology was limited to the ceca and consisted of severe hemorrhage and tissue damage. At 5 days post-infection histological examination revealed larger second generation schizonts in the subepithelium of the ceca. Mortality was common with severe infections (15,000 oocysts). Single oocyst cloning was periodically done to insure purity of the E. tenella isolate.

Propagation of Oocysts. Pure cultures of this isolate were routinely passaged in 4- to 6-week old SPF white leghorn chickens. To avoid extraneous coccidial infections, chickens were reared from 1 day of age in plexiglass isolation units. Oocysts were harvested on day 7 post-infection from the ceca using a trypsin-digest method described by Shirley (48). Sporulated oocysts were typically stored at 24°C in 2% w/v $K_2Cr_2O_7$.

Isolation of Sporocysts. Sporulated Eimeria tenella oocysts, ($1 \times 10^8$) which had been partially purified from debris by salt floatation, were washed five times in 0.1M phosphate buffered saline, pH 7.4, (PBS) to remove the potassium dichromate preservative. These oocysts were further cleaned by agitation in a 1.05% sodium hypochlorite solution for 15 minutes followed by five washes in PBS to remove residual sodium hypochlorite and debris. Following the final wash, the cleaned oocysts were resuspended in 10 ml of PBS. Suspended oocysts were then mechanically broken by shaking with an equal volume of glass beads (1.0-1.05mm). The liberated sporocysts were purified from the oocyst walls and from unbroken oocysts by passage over a glass wool column, centrifuged at 10,000 RPM for ten minutes at 4°C and resuspended in 10 ml of PBS.

Preparation of Sporozoites. Recently sporulated oocysts were cleaned by salt floatation, repeated washing and treatment with 1.05% sodium hypochlorite solution. Sporocysts were freed by mechanically breaking oocysts with glass beads (1.0-1.05mm). To excyst sporozoites, sporocysts were incubated with trypsin and taurodeoxycholic acid (0.25 and 0.50% w/v, respectively) for a period of 1 hour at 41°C. Sporozoites thus obtained were rinsed free of excysting fluid by centrifugation and resuspended in Hank's medium. Fresh Hank's medium was used to dilute sporozoites to the working concentration.

## EXAMPLE 2

## GENERATION, IDENTIFICATION AND CHARACTERIZATION OF HYBRIDOMAS

### Monoclonal Antibody.

Monoclonal antibodies were derived from hybridomas developed using the method of VanDeusen and Whetstone (55). Briefly, Balb/C ByJ mice were repeatedly immunized with $10^6$-$10^7$ intact E. tenella sporozoites. Three days after a final intravenous injection with intact sporozoites, a randomly selected mouse was sacrificed and splenectomized. The splenocytes were separated from fibrous tissue in the organ, and the washed cells fused with the murine plasmacytoma cell line SP2/OM.

### Microneutralization Assay.

The microneutralization assay was performed with primary chick kidney cell cultures. 1- to 2-week-old chicks were sacrificed and aseptically nephrectomized. The kidneys were trypsinized, and cells plated into 96 well cultures at a density of approximately $10^4$/well in Earle's LAH medium supplemented with 5% heat-inactivated fetal calf serum. Cultures were maintained at 41°C in a 5% $CO_2$ atmosphere. When cell cultures reached a level of approximately 50% confluency, 50 $\mu$l of hybridoma test or control sample were added to all wells of the plate. Next, 2.3 x $10^4$ sporozoites in 50 $\mu$l of Earle's culture medium were added to all wells of the plate. Twelve to sixteen hours later, the culture supernatant was replaced with fresh Earle's LAH containing 2% heat inactivated fetal calf serum. The cultures were terminated at 40-44 hours post infection. Culture supernatant was emptied from the plates at that time. Subsequently, cells were fixed to the plates by the addition of methanol acidified with 5% glacial acetic acid. The fixed cultures were stained with 0.1% toluidine blue before examination. Wells were scored as to the approximate percentage level of inhibition of schizogony.

Indirect Fluorescent Antibody Screening

IFA slides were prepared with sporozoites of E. tenella (about 1x10$^6$/well). Slides were air dried overnight before 10 $\mu$l of 1% BSA was added to each well. Five minutes after adding BSA, 20 $\mu$l of test supernatant was added. Supernatants were incubated at 37°C for 20 minutes, followed by three rinses with 0.15M PBS with 0.0005% Tween-20 (PBS-Tween). Fluorescent conjugated rabbit anti-mouse antibody (diluted 1:40 in PBS) was added to the samples and allowed to incubate at 37°C for 20 minutes. The conjugate was rinsed off three times with PBS-Tween before adding mounting medium and cover slip.

Results.

Of the several thousand hybridomas developed against Eimeria tenella, 24 were found to produce neutralizing antibodies toward the sporozoite stage of the parasite. All of the hybridomas studied produced antibodies that recognized membrane bound antigens, although only the antibody produced by one hybridoma recognized an internal membrane antigen. The other monoclonal antibodies examined recognized antigens associated with the plasmalemma.

In vitro neutralizing potency was compared for several supernatants after the initial cloning of the respective cell lines. Supernatants from two lines demonstrated the greatest relative propensity for neutralizing sporozoites of E. tenella. When antibody content was assessed for each of supernatants tested, it was determined that twenty-fold less of one antibody (designated Ptn 7.2A4) was required to neutralize sporozoites than the second most effective neutralizing antibody. Specifically, the amount of Ptn 7.2A4 antibody required to neutralize E. tenella is approximately 3.5 x 10$^5$ molecules/sporozoite.

EXAMPLE 3

IDENTIFICATION OF THE ANTIGENS OF E. TENELLA RECOGNIZED
BY BOTH NEUTRALIZING MONOCLONAL ANTIBODY A4 AND
E. TENELLA SPOROZOITE-SPECIFIC CHICKEN ANTI-SERUM

$^{125}$I labeling of Eimeria proteins.

A total of 2x10$^8$ oocysts from E. tenella were processed for iodination. In each case, sporocysts were purified from salt floated, sodium hypochlorite treated oocysts that were broken with glass beads then passed through a glass wool column. Sporocyst membranes were prepared from one-half of the sporocysts by mechanical breakage in 1 ml 10 mM sodium phosphate, 0.15M NaCl, pH 7.2 (PBS) with glass beads in the presence of protease inhibitors: 0.1mM Phenylmethylsulfonyl fluoride (PMSF), 0.1 mM N-tosyl-L-phenylalanine chloromethyl ketone (TPCK), 1 mM N-$\alpha$-p-Tosyl-L-Lysine chloromethyl ketone (TLCK) and 10 KIU/ml aprotinin. The remaining sporocysts were treated with trypsin and taurodeoxycholic acid (total volume = 1 ml) to excyst sporozoites. Both preparations were pelleted at 45,000 RPM for 45 minutes at 4°C and resuspended in 1 ml of phosphate buffered saline (pH 7.4) (PBS). Care was taken to remove all trypsin - deoxycholate residue from the sporozoites by washing with PBS and 1 mM PMSF prior to ultra-centrifugation.

The one ml samples were put into glass scintillation vials which had been coated with 40 $\mu$g of IODOGEN (1,3,4,6 - tetrachloro-3$\alpha$, 6$\alpha$-diphenylglycouril) solid phase iodination reagent, dried under nitrogen gas and rinsed with PBS. To each tube, 0.5 mCi of $^{125}$I was added and the samples allowed to incubate for 20 minutes on ice. Afterward, 100 $\mu$l of KI (1 M) was added to each tube to a final concentration of 100 mM, and the reaction was allowed to proceed for an additional 15 minutes on ice. Sporozoite and sporocyst preparations were then diluted to 7 ml with PBS containing 5 mM KI and pelleted at 45,000 RPM for 45 minutes at 4°C.

Extraction of Sporocyst and Sporozoite Membrane Proteins.

$^{125}$I labeled sporocyst and sporozoite pellets from the above high speed centrifugation were resuspended in 1 ml of protein extraction buffer (20 mM Tris-HCl, pH 7.5; 50 mM MgCl$_2$; 25 mM NaCl, 1% NP40, 1 mM PMSF, 0.1 mM TPCK, 1 mM TLCK and 10 KIU/ml aprotinin). The suspensions were incubated for 30 minutes on ice with occasional vortexing. Insoluble material was separated from the detergent solubilized protein in a microfuge for 15 minutes at 4°C. The supernatants were stored overnight at -70°C.

TCA Precipitation of $^{125}$I Proteins.

Ten microliters of each sample were diluted into 90 $\mu$l of 5 mM KI. Ten microliters of each diluted sample was then added to a solution containing 1 ml of 5% trichloroacetic acid (TCA), 25 $\mu$l BSA (10 mg/ml) and 5 mM KI and incubated on ice for 30 minutes. The precipitated samples were collected by filtration through glass fiber filters, washed twice with 5 ml of 5% TCA, 5 mM KI and three times with 5 ml of 95% ethanol, both at 0°C, and counted in a scintillation counter.

SDS-Polyacrylamide Gel Electrophoresis (SDS-PAGE) of Total $^{125}$I labeled E. tenella Fractions.

Total $^{125}$I labeled sporocyst, sporozoite and soluble membrane proteins and immunoprecipitated proteins (see below) were analyzed on 120x100x0.75 mm, 5-25% exponential gradient SDS-polyacrylamide gels at 20 mA for 3 hours. The gels were dried and used to expose x-ray film at -70°C.
Gels used for staining purposes were visualized by silver staining.

Immunoprecipitations With Monoclonal Antibody.

Fifty microliters of hybridoma supernatant containing monoclonal antibody were added to 25 $\mu$l of monoclonal antibody dilution buffer (MAB-DIL) (50 mM Tris-HCl, pH 8.6; 150 mM NaCl; 0.1% NP40; 0.1% BSA, RIA grade; 1 mM TLCK; 1 mM PMSF, 10 KIU/ml aprotinin). Twenty microliters of $^{125}$I labeled protein was then added and the tube was vigorously mixed and then incubated overnight at 4°C. Rabbit anti-mouse Ig serum (IgA, IgG, IgM reactive) was diluted 1:2 in MAB-DIL and 10$\mu$l added to each immunoprecipitation tube and incubated 1 hour at 4°C. Protein A-Sepharose (10% v/v) was diluted 1:4 in monoclonal antibody wash buffer, pH 8.6 (MABW) (50 mM Tris.HCl, 0.05% NP40, 0.05% Triton X-100, 150 mM NaCl, 0.02% NaN$_3$, 5 mM KI) and 400 $\mu$l added to each tube. The tubes were incubated for one hour at 4°C with gentle rocking. The immunoprecipitation products were washed twice with cold MABW followed by two room temperature washes with MABW. The pellet was resuspended in 50 $\mu$l of 1.5X SDS-PAGE sample buffer (24) boiled for 5 minutes and microfuged to remove the sepharose sorbant. Supernatants were counted as to total radioactivity and analyzed by SDS-PAGE.

Immunoprecipitation With E. tenella Sporozoite Specific Chicken Anti-serum.

The same procedure was used as described in the section concerning immunoprecipitation with monoclonal antibodies with a few exceptions. Sera were diluted 1:10 in 90 $\mu$l of antibody dilution buffer (AB-DIL) (10 mM Tris-HCl, pH 7.5; 0.1% BSA RIA grade; 1 mM PMSF; 1 mM TLCK; 10 KIU/ml aprotinin) prior to the addition of 20 $\mu$l of $^{125}$I labeled protein. The antibody wash buffer (ABW) consists of 10 mM Tris-HCl, pH 7.5, 0.1% BSA RIA grade; 1 mM PMSF; 1 mM TLCK, 10 KIU/ml aprotinin; 0.05% NP40; 0.05% Triton X-100, ± 5 mM KI.

Results of Immunoprecipitation of E. tenella Antigens with Ptn 7.2A4/4 Monoclonal Antibody.

The surface-labeled E. tenella sporozoite preparation is enriched for two major, iodinated polypeptides, one at 6,500 daltons and one at 25,000 daltons. The 6,500 dalton peptide has previously been determined to be a protease digestion fragment of the 17,000 molecular weight polypeptide found in the sporocyst preparation and is readily and specifically immunoprecipitated with monoclonal antibody Ptn 7.2A4/4. Membranes from sporocysts are enriched for two major iodinated polypeptides of 17,000 and 27,000 daltons although several other minor proteins of various molecular weights are also present. Upon immunoprecipitation of $^{125}$I-labeled sporocyst membrane protein, the only antigen recognized by the monoclonal antibody Ptn 7.2A4/4 is the 17,000 molecular weight polypeptide whose amino acid sequence is described in Example 5.

Results of Immunoprecipitation of E. tenella Antigens With E. tenella Sporozoite Specific Chicken Anti-sera.

Sera from chickens immunized by repeated intravenous injections of killed E. tenella sporozoites were used to immunoprecipitate E. tenella proteins in order to determine if they recognize the 17,000 dalton polypeptide as does monoclonal antibody from hybridoma cell line HB8561.

From the iodinated sporocyst and sporozoite membrane preparations, the 17,000 and 6,500 dalton polypeptides, respectively, are specifically immunoprecipitated by anti-sera from chickens immunized as described above. Sera from specific pathogen-free control chickens do not appear to recognize any E. tenella proteins.

Results of Western Blots of E. tenella Antigens with Ptn 7.2A4/4 Monoclonal Antibody.

Under the conditions in which the immunoprecipitated, iodinated polypeptides were analyzed on SDS-PAGE as described above, polypeptides linked by disulfide bonds have been separated. However, reduction of disulfide bonds destroys A4 reactivity on Western blots in both sporocyst and sporozoite membrane preparations. When iodinated sporocyst and sporozoite membrane preparations were run on SDS-PAGE under non-reducing conditions the major radiolabeled species migrates with an apparent molecular weight of 21-23,000. Furthermore, this 21-23,000 dalton species was reactive with monoclonal antibody A4 by western blotting (Vectastain ABC Kit for Mouse IgG, Vector Labs, Burlingame, CA). These results suggest that the 17,000 dalton polypeptide in sporocyst membranes and the 6,500 dalton polypeptide in sporozoite membranes are complexed to another polypeptide(s) in the A4 antigen. The fact that this other polypeptide component of the A4 antigen was not observed in immunoprecipitation experiments of iodinated material can be explained by the observation that this other polypeptide does not contain any tyrosines that could be iodinated (see description of the 8,000 dalton polypeptide component of the A4 antigen in Examples 5 and 6).

EXAMPLE 4

# PURIFICATION, IDENTIFICATION AND CHARACTERIZATION OF THE EIMERIA TENELLA A4 ANTIGEN AND FRAGMENTS THEREOF

Purification of the 17,000 dalton peptide component of the A4 Antigen.

E. tenella sporulated oocysts were resuspended in 10 ml PBS per $10^9$ oocysts and were broken by shaking with an equal volume of glass beads. Membranes were isolated by centrifugation (100,000 x g, 60 min., 4°C) and the proteins were solubilized in 1% NP-40, 10 mM Tris (pH 7.5), 25 mM NaCl, 1 mM PMSF, 1 mM TLCK, 0.1 mM TPCK and 10 KIU/ml aprotinin. Insoluble material was pelleted with another 100,000 x g spin (60 min., 4°C). The protein was adsorbed to a DEAE-cellulose column equilibrated with 10 mM Tris (pH 7.7), 0.05% NP-40 and then washed with this buffer containing 50 mM NaCl. After elution with buffer containing 200 mM NaCl, the 17,000 dalton polypeptide was concentrated by acetone precipitation and the precipitate resuspended in loading buffer, boiled and subjected to electrophoresis in SDS-polyacrylamide (15%). Conventional SDS-PAGE sample buffer used in this and other experiments contained 62.5 mM Tris-HCl (pH 6.8), 2% (w/v) sodium dodecyl sulfate, 10% (w/v) glycerol and 0.001% (w/v) bromphenol blue. The buffer also contained 5% (w/v) $\beta$-mercaptoethanol except in SDS-PAGE experiments in which non-reducing conditions are specified. The 17,000 dalton polypeptide band was identified by staining (Coomassie blue or KCl). The appropriate gel region was excised, the protein electroeluted and concentrated by acetone precipitation. Note that these procedures are denaturing for proteins and peptides bound to each other by disulfide bonds are separated with this method. The 17,000 dalton polypeptide purified by this method was essentially pure.

Purification and Characterization of the A4 Antigen.

Alternatively to purification by gel electrophoresis the sporocyst membrane proteins from the DEAE-cellulose column were dialyzed against 10 mM Tris HCl, pH8, 0.05% NP-40 and applied to a DEAE-HPLC column (BioRad) equilibrated in this buffer. The column was developed with a NaCl gradient (0-300 mM) in the same buffer. The 17,000 dalton polypeptide (identified by its migration on gel electrophoresis) was found in material eluting at 200 mM NaCl. Fractions containing this protein were applied to a hydroxylapatite column (HPHT-BioRad) equilibrated with 30 mM potassium phosphate, pH 6.5, 0.05% Zwittergent 3-12 (Calbiochem-Behring, LaJolla, CA) 0.1 mM dithiothreitol. The column was washed with equilibration buffer and developed with a potassium phosphate gradient (0-300 mM) containing 0.05% Zwittergent and 0.1 mM dithiothreitol. The 17,000 dalton polypeptide (identified by gel electrophoresis described above) appeared in material eluting at approximately 90 mM potassium phosphate.

The fractions containing the 17,000 dalton polypeptide purified by this method also contained a second peptide of 8,000 daltons. This peptide appears to be linked by a disulfide bridge to the 17,000 dalton polypeptide. If the fractions containing the 17,000 dalton peptide were immuno-precipitated with monoclonal antibody A4 and the precipitated proteins were analyzed by gel electrophoresis under reducing conditions (as above) both the 17,000 and 8,000 dalton polypeptides appear to be immunoprecipitated. Hence in sporocyst membrane preparations the 8,000 dalton and 17,000 dalton polypeptides appear to be linked by a disulfide bond (presumably by a cysteine bridge) because the two peptides did not appear on electrophoresis unless a strong reducing agent was present. Under non-reducing conditions the A4 reactive species migrates with an apparent molecular weight of 21-23,000.

Preparation of the 11,500 dalton fragment of the A4 antigen.

E. tenella sporocyst membranes were prepared as described above and resuspended in 10 ml of PBS + 1% Triton X -100. To this 10 ml membrane suspension was added 10 ml of 80% phenol containing 0.1% 8-hydroxyquinoline. The suspension was then vortexed at maximum speed for three minutes and centrifuged for ten minutes at 4000 RPM. The phenol and the flocculent interface were removed and diluted in five volumes of 100 mM ammonium acetate in methanol and allowed to precipitate at -20°C overnight. Following two washes in acetone, the insoluble proteins were agitated for 8 hours in 0.5% SDS, and insoluble materials removed by centrifugation at 20,000 RPM for one hour at 4°C. The sample was dialyzed extensively against PBS (pH 7.2) containing AG 501-X8 mixed bed resin (1 gm/500 ml). The 11,500 dalton fragment of the A4 antigen was then immunoadsorbed from the supernatant using the Ptn 7.2A4/4 monoclonal antibody. This polypeptide was shown to be reactive with the Ptn 7.2A4/4 monoclonal antibody by microtiter plate ELISA.

For microtiter plate ELISA polystyrene 96 well clusters (Immulon II) were sensitized with antigen in 10 mM glycine buffered saline, pH 9.6, incubated overnight at 37°C. The wells were washed with 0.15M PBS with 0.0005% Tween-20, blocked with 3% BSA in PBS-Tween, rewashed, and incubated with Ptn 7.2A4/4 monoclonal antibody diluted in PBS. The wells were washed as before, and then incubated with peroxidase conjugated rabbit anti-mouse IgG serum diluted in PBS. The wells were washed again and then incubated with substrate (2,2'-azino-di-[3-ethyl-benzthiazoline sulfonate]) in the presence of $H_2O_2$. Color development was determined with a Dynatech MR-580 microtiter plate ELISA reader after 15 minutes.

Preparation of the 6,500 dalton fragment of the A4 antigen.

E. tenella sporocysts were prepared. Sporocysts were then incubated for 1 hour at 41°C in a solution containing 0.25% trypsin and 0.5% taurodeoxycholic acid in order to liberate sporozoites. Sporozoites were then washed several times in PBS containing 1 mM PMSF and sporozoite membranes prepared by mechanical breakage of sporozoites with glass beads. The 6,500 dalton fragment of the A4 antigen could be immunoprecipitated with the Ptn 7.2A4/4 monoclonal antibody or the E. tenella sporozoite-specific chicken antisera.

Relationship of the Various Peptides and Fragments of the A4 Antigen.

As will be shown in Examples 5 and 6 the gene encoding the A4 antigen encodes a 25,000 dalton protein. This protein is subsequently processed by proteolysis to produce 17,000 and 8,000 dalton peptides which are linked by a disulfide bond. Both the 11,500 and 6,500 dalton fragments of the A4 antigen are believed to be fragments of the 17,000 dalton peptide. Since the 6,500 dalton fragment labels well with iodine whereas the 8,000 dalton peptide does not iodinate we conclude the 6,500 dalton fragment is derived from the 17,000 dalton peptide. The 11,500 dalton fragment is seen on reducing SDS-PAGE and therefore must be derived from the 17,000 dalton peptide. The exact location of these two fragments within the 17,000 dalton peptide is unknown.

EXAMPLE 5

AMINO ACID SEQUENCE OF THE 17,000 AND 8,000 DALTON PEPTIDE COMPONENTS OF THE A4 ANTIGEN

Amino Acid Sequence of the 17,000 dalton peptide component of the A4 Antigen.

Amino acid sequencing of the 17,000 dalton peptide was complicated by the finding that the N terminal amino acid was blocked (i.e. not accessible to Edman degradation (12)). To circumvent this problem the protein was reduced and alkylated and then digested with various chemicals and enzymes. The resulting peptides were purified by reverse phase HPLC (17). The 17,000 dalton polypeptide or the A4 antigen was digested with CNBr (CN), V8 protease (V), chymotrypsin (CH) and Endoprotease Arg-C (R).

Before protease digestion the purified 17,000 dalton polypeptide or the A4 antigen was treated with 30 mM dithiothreitol, 6M guanidine-HCl (pH 8) for 1 hour at room temperature. Solid iodoacetamide was added to a final concentration of 100 mM, the pH was readjusted to 8 and the sample was incubated for 1 hour at room temperature. Following reduction and alkylation samples were purified from reagents either by P6DG (Bio-Rad, Richmond Cal.) spin columns equilibrated in 0.1M MOPS, pH 7.5, 0.1% SDS or by reverse phase HPLC.

For CNBr digestion the protein sample was treated with 1% CNBr in 70% formic acid for 20 hours at 4°C. The sample was evaporated to dryness in a Savant Speedvac centrifuge and redissolved in 0.1% trifluoroacetic acid (TFA) or 0.1% TFA, 20% acetonitrile ($CH_3CN$). V8 digestion was performed in 0.1% SDS, 0.1M MOPS pH 7.5 for 2 hours at room temperature at a ratio of 50 $\mu$g 17,000 dalton polypeptide: 1$\mu$g V8. After digestion the samples were precipitated with 4 volumes of acetone at -20°C overnight. The acetone precipitates were redissolved as described above. Chymotrypsin digestion was performed in 0.05% Zwittergent 3-12, 0.1M $NH_4HCO_3$, pH 7.8 for 1 hour at 37°C at a ratio of 50:1, 17,000 dalton peptide:chymotrypsin. Samples were acidified with TFA for peptide purification. Arg-C digestion was performed in 0.05% Zwittergent 3-12, 0.1M $NH_4HCO_3$ pH 7.8 for 2 hours at 37°C at a ratio of 15:1, 17,000 dalton peptide: Arg-C. After acetone precipitation overnight at -20°C the peptides were mainly in the acetone supernatant. The supernatant was evaporated and the samples redissolved as described above. Peptides were purified on a Vydac C4 column (the Separations Group, Inc., Hisparia, CA) and eluted with a 0-100% $CH_3CN$ gradient in 0.1% TFA.

Amino acid sequencing was performed using a gas phase sequencer (Applied Biosystems, Inc., Foster City, CA) according to the procedure of Hunkapiller et al. (16). Phenylthiocarbamyl (PTC) derivatized amino acids were analyzed by HPLC (5).

The N terminal amino acid was determined directly by removing the blocking agent. The 17,000 dalton peptide was treated with pyroglutamate aminopeptidase (5:1 protein:PAP) in 0.1M potassium phosphate (pH 8.0), 10 mM EDTA, 5% glycerol, 5 mM dithiothreitol, 0.05% Zwittergent 3-12 for 1 hour at 37°C. After treatment the amino acid sequence could be determined directly suggesting that the N terminal amino acid glutamine is cyclized to form the blocked residue pyrrolidone carboxylic acid.

The complete amino acid sequence for the 17,000 dalton peptide component of the A4 antigen is shown in Figure 1.

Partial Amino Acid Sequence of the 8,000 dalton Peptide Component of the A4 Antigen.

When the purified 8,000 dalton peptide (derived from the A4 antigen by reduction and alkylation) was subjected to Edman sequencing the N terminal amino acid sequence could be determined directly. A partial amino acid sequence of the N terminal region of the peptide is shown below:

```
NH2 - ala  ala  gly  thr  thr  asp  ala  val  ile  cys
      leu  thr  asn  pro  ala  pro  leu  glu  ala
      arg  ser  gln  pro  phe  asp  asp  glu
```

EXAMPLE 6

ISOLATION AND CHARACTERIZATION OF A GENOMIC DNA CLONE ENCODING THE A4 ANTIGEN

Isolation of DNA from E. tenella Sporulated oocysts.

Sporulated oocysts ($5 \times 10^8$) were washed and sporocysts were isolated as described previously. Isolated sporocysts were washed 2X with 0.1M Tris-HCL, (pH 8.5), 0.2M NaCl, 10mM EDTA. Sporocysts were lysed by incubation for 30 min. at 65°C in 0.1M Tris-HCl, (pH 8.5), 0.2M NaCl, 50 mM EDTA, 1% SDS, 150 $\mu$g/ml Proteinase K. After cooling to room temperature the DNA was gently extracted with an equal volume of phenol for 1 hour. After centrifugation for 10 min. at 3,000 rpm the aqueous layer was removed and the interface and phenol were reextracted with 10 mM Tris-HCl (pH 8), 1 mM EDTA. The aqueous phases were pooled and extracted 1X with phenol and 2X with chloroform: isoamyl alcohol (24:1). DNA was isolated by ethanol precipitation. The DNA pellet was redissolved in 10mM Tris-HCl (pH 8), 1 mM EDTA and treated with 0.15 mg/ml DNase free-RNase A for 1 hour at 37°C. After RNase digestion the sample was extracted 1X with phenol, 1X with chloroform: isoamyl alcohol and then precipitated with ethanol. On agarose gels the size of the DNA was determined to be greater than 20 kilobase pairs.

Construction of the E. tenella Genomic Library in Bacteriophage λgt wes λB.

The E. tenella genomic DNA library in bacteriophage λgt wes λB (25) constructed using methods described by Maniatis et al. (32). Phage were purified by polyethyleneglycol precipitation, chloroform extraction and CsCl gradient centrifugation. Purified phage were disrupted with 1% SDS, 50mM EDTA and 150 $\mu$g/ml Proteinase K, and DNA was purified by phenol extraction, chloroform extraction and ethanol precipitation. The E. tenella genomic DNA and phage DNA were digested to completion with EcoRI. The left and right arms of the phage DNA were annealed at their cohesive ends and the arms were purified by sucrose density gradient centrifugation. 30 $\mu$g EcoRI digested DNA arms were ligated to 6$\mu$g EcoRI digested E. tenella DNA using T4 DNA ligase. 20 $\mu$g of the ligated DNA was packaged in vitro into phage particles producing a library of $5 \times 10^6$ recombinant phage particles.

Synthetic oligonucleotides

Oligonucleotide probes that would be complementary to regions of the gene encoding the 17,000 dalton peptide component of the A4 antigen were synthesized using a Biosearch Sam I (Biosearch, Inc., San Rafael, CA). The expected DNA sequences of the appropriate regions were deduced from the amino acid sequence of the 17,000 dalton peptide. Because of the ambiguity in the genetic code, the exact DNA sequence cannot be predicted. "Mixed probes" were designed and synthesized which contained a mixture of DNA sequences, one of which should have perfect match homology with the gene for the 17,000 dalton peptide.

Oligonucleotide COD 92 was based on amino acids 6 to 12 of peptide VI (see Example 5 for the amino acid sequence of the 17,000 dalton peptide). It contained a mixture of 256 different sequences. The structure of oligonucleotide COD 92 is:

```
                G   A   A   G   A   A
3' - CCATT AA CGAAT AT GG - 5'
        T   G   G   T   G   G
        C               C
```

```
Amino Acid
Sequence:      GlyAsnPheAlaTyrTyrPro
```

Oligonucleotide COD 94 was based on amino acids 3 to 8 of peptide V2 of the 17,000 dalton peptide. It contained a mixture of 64 different sequences:

```
              A   G   A   G
5' - TGGAA ACAGA ATATG - 3'
          G   T   G   T
          C       C
```

```
Amino Acid
Sequence:      TrpLysThrGluIleCys
```

Oligonucleotide COD 108 was based on amino acids 25-30 of peptide V1. It contained a mixture of 16 different sequences. The structure of oligonucleotide COD-108 is:

$$3' - CT_{C}^{T}AT_{A}^{G}ACCTT_{C}^{T}CC_{A}^{G}CC - 5'$$

**Amino Acid**

**Sequence:**        **GluTyrTrpLysGlyGly**

Screening the E. tenella Genomic DNA Library.

Recombinant phage of the E. tenella genomic DNA library were plated on 15 cm plates at high density, up to 2-3 X 10⁴ phage per plate. Nitrocellulose filter replicas of each plate were prepared according to the method of Benton and Davis (2). The filters were then incubated with the appropriate synthetic oligonucleotides which had been labeled to high specific activity with ($^{32}$P)-ATP and T4 polynucleotide kinase. Positive plaques were identified by autoradiography. Only those plaques that hybridized to both oligonucleotides COD-92 and 108 were scored positive.

Small blocks of agar were cut from the plates in regions that corresponded to the region of the filter containing the hybridizing DNA. The phage were eluted, replated at lower density (20-100/plate) and rescreened with all three oligonucleotide probes. Pure isolated positive plaques or clones were picked. Phage 108-1 hybridized strongly to oligonucleotide COD-92 and moderately to oligonucleotides COD-108 and 94. Phage 108-1 was grown up on a larger scale for purification and characterization of the E. tenella DNA insert. Characterization of phage 108-1 DNA showed an EcoRI insert of 5,500 bp.

Detailed Characterization of the Genomic Clone Encoding the 17,000 dalton Peptide - Restriction Map.

The 5,500 bp Eco RI fragment insert of clone 108-1 was subcloned from the λphage vector into plasmid pUC 9 (56). The recombinant plasmids were digested with a variety of restriction endonucleases to determine the position of key restriction sites in the genomic DNA clone. The position of restriction sites within the DNA was needed to determine the location and orientation of the 17,000 dalton peptide gene and to develop a strategy to sequence the Eco RI genomic DNA fragment. The restriction map is presented in Figure 2. The location and orientation of the gene for the 17,000 dalton peptide is shown on this map.

DNA Sequence Analysis of Clone 108-1.

The BgIII-Eco RI fragment of clone 108-1 containing the gene for the 17,000 dalton peptide component of the A4 antigen was sequenced by the dideoxy method of Sanger (44) using various restriction enzyme fragments. Primers for DNA synthesis included oligonucleotides COD-92, 94 and 108 as well as other synthetic oligonucleotides. The DNA sequence is shown in Figure 3.

Structure of the Gene Encoding the A4 Antigen.

The DNA sequence agrees with that predicted by the amino acid sequence analysis. In addition there are three features of the gene which are not apparent from the protein sequence. Using protein sequence information and general information regarding the structure of secretory proteins we have deduced the structure of the gene for the A4 antigen.

From the known amino terminus of the sporocyst membrane 17,000 dalton peptide (see Example 5), Gln-Asp-Tyr---, we see that the gene encodes an extra 23 amino acids upstream. This DNA sequence is a typical "signal" sequence found at the amino terminus of genes for many secretory or membrane proteins (23, 3). The peptide it encodes is required for the export of proteins from their site of synthesis (the cytoplasm) to and/or through the plasma membrane. The signal peptide is usually removed during the secretory process. It is not surprising that the A4 antigen is made with a signal peptide since it most likely traverses the cytoplasmic membrane in order to be found at the outer surface of the sporozoite. The amino terminus of the signal sequence is assumed to be the Met codon since, essentially, synthesis of all proteins begin with methionine.

There are three regions of the gene in which the DNA sequence does not coincide with the protein sequence. The first is a 101 bp segment occurring within the codon for Val-7 of the known mature 17,000 dalton protein sequence. The second is a 114 bp sequence between the codons for Gly-65 and Gly-66 of the 17,000 dalton peptide. The third is a 124 bp sequence within the codon for Asp-186 of the 8,000 dalton peptide. These three sequences are intron structures typically found within the coding regions of many eukaryotic genes. They are present in the precursor to the mRNA, and then removed by an RNA recombination mechanism known as "splicing," to give the mature mRNA with an uninterrupted coding sequence. The DNA sequences around the "splice junctions" are consistent with those seen in other eukaryotic genes (47).

The sequence of the 17,000 dalton peptide appears to terminate with the sequence Gly-Gly corresponding to codons 157 and 158. We have also identified an 8,000 dalton peptide with the sequence beginning with Ala-162 extending to Glu-188. The peptide sequence Arg-Arg-Leu corresponding to codons 159 through 161 has not been found. It is probable that this tripeptide is removed by a mechanism similar to the cleavage of other proteins such as insulin (53). Hence the two peptides of the A4 antigen are encoded by a contiguous nucleotide sequence, and at least one proteolytic step occurs to generate the 8,000 dalton peptide beginning with Ala-162.

EXAMPLE 7

PREPARATION OF ANTI-IDIOTYPE ANTIBODY TO THE A4 PROTEIN ANTIGEN

The A4 anti-idiotype antibody was prepared by sensitizing rabbits with purified A4 monoclonal antibody.

Preparation of A4 Monoclonal Antibody. Ptn 7.2A4/4 hybridomas were propagated in HB101® serum free media (HANA Biologics) to a density of approximately $2 \times 10^6$ cells/ml. Supernatants were tested by the double radial immunodiffusion method in agarose gels against anti-mouse IgG and anti-mouse $\gamma$G3. Positive supernatants were pooled and concentrated approximately 50-fold by ultrafiltration and dialyzed against 50 mM Tris, 150 mM NaCl, 0.2% $NaN_3$, pH 8.4. The supernatant was then passed over a Protein A - Sepharose column to bind immunoglobulin. The column was washed with 15-bed volumes of the above buffer to remove all traces of unbound protein. Bound antibody was eluted from the column in 2 ml fractions with 100 mM sodium acetate, 0.04% sodium azide, pH 4.0, into tubes containing 2 ml of 500 mM phosphate buffer, pH 7.5. The antibody was then passed through a DEAE Affi-gel® blue column (Bio-Rad Labs) and peak fractions pooled and concentrated by ammonium sulfate precipitation. The concentrated antibody was dialyzed to remove residual salt and quantitated by the Lowry protein quantitation method. Purity was determined by electrophoretic separation in 8-20% SDS-PAG polyacrylamide gel.

A4 Anti-Idiotype Antibody Production. Rabbits were immunized with an initial dose of 500 $\mu$g purified Ptn7.2A4/4 antibody in Complete Freunds Adjuvant followed by three booster administrations of 500 $\mu$g of antibody each in Incomplete Freunds Adjuvant, ten days apart. Rabbits were bled by cardiac puncture and sera prepared and immediately stored at -70 C.

Preparation of Mouse Serum Protein Immunoadsorption Column.

Non-immune mouse serum was ammonium sulfate (50% saturated) precipitated and the precipitated proteins resuspended in PBS and passed over a Sephadex G-25 column to remove residual salt. Peak fractions were pooled and dialyzed against 100 mM $NaHCO_3$, 500 mM NaCl, pH 8.3 and covalently bound to CNBr-activated Sepharose.

Purification of A4 Anti-Idiotype Antibody. Immune rabbit serum against Ptn 7.2A4/4 was loaded onto the immunoadsorption column prepared with mouse serum proteins. Unbound antibody was collected in 2 ml fractions and tested for activity against Ptn7.2A4/4 by double radial immunodiffusion. Positive fractions were then examined for antibody heavy and light chains by SDS-PAGE. This preparation can be used to vaccinate chickens. A typical protocol for vaccination/protection studies may be as follows. Birds may be vaccinated intramuscularly with 100 $\mu$g of A4 anti-idiotypic antibody adjuvanted with three parts carrier consisting of 5% Arlacel 4, 94% Drakeol 6-VR and 1% Tween-80. Control birds may either be unvaccinated or receive adjuvanted antibody from control rabbits purified in the same way as the A4 anti-idiotype antibody. Fourteen days later the birds may be revaccinated with another 100 $\mu$ g of adjuvanted antibody. Fourteen days after the second vaccination the birds may be challenged with 12,000 E. tenella oocysts. The birds may be lesion scored five days post-challenge. Lesions scores may be reduced 50-60% in A4 anti-idiotype antibody vaccinated birds when compared to controls.

## EXAMPLE 8

### PASSIVE PROTECTION OF CHICKENS USING MONOCLONAL ANTIBODY PTN 7.2A4/4

Protection against E. tenella and E. necatrix Sporozoite infection using monoclonal antibody Ptn.7.2A4/4. Two experiments were done to determine if monoclonal antibody Ptn 7.2A4/4 developed against E. tenella sporozoites and shown to neutralize E. tenella sporozoites in vitro, could passively protect chickens from an infection of E. tenella or E. necatrix sporozoites via cloacal or oral routes respectively. In experiment 1, six groups of seven 4-week-old SPF white Leghorn chickens were inoculated with either $10^6$, $10^5$ or $10^4$ sporozoites of E. tenella intracloacally. Sporozoites were preincubated for one hour in eight times the minimal neutralizing dose of Ptn 7.2A4/4 monoclonal antibody ($1.75 \times 10^6$ antibody molecules/sporozoite) or an equal volume of SP2/0 myeloma supernatants. In experiment 2, six groups of seven 4-week-old SPF white Leghorn chickens were inoculated orally with $10^6$, $10^5$, or $10^4$ E. necatrix sporozoites. Prior to inoculation, sporozoites were incubated for a period of one hour in 5 times the minimal neutralizing dose of Ptn7.2A4/4. Control sporozoites were incubated in SP2/0 myeloma culture supernatants.

Intracloacal and oral inoculation with sporozoites was done using syringe and coagulation tip. The appropriate number of sporozoites was given in either 0.5 or 1 ml dose.

In experiment 1, three birds died of coccidiosis between days 4 and 5 in the control group receiving $10^6$ sporozoites. There was one mortality in the treatment group. Likewise, lesion scores and hematocrit readings indicated a more severe infection in the control groups than in the treatment groups.

| IN VIVO NEUTRALIZATION OF E. TENELLA SPOROZOITES | | | | |
|---|---|---|---|---|
| Sporozoite Treatment | Sporozoite Dose* | Hematocrit** $\bar{x}$ ± s.d. | Lesion Score $\bar{x}$ ± s.d. | No. of Deaths |
| Control | $10^4$ | 30.1 ± 1.9 | 2.4 ± 0.7 | 0 |
| Control | $10^5$ | 23.4 ± 6.8 | 3.0 ± 0.8 | 0 |
| Control | $10^6$ | 20.4 ± 5.7 | 3.9 ± 0.3 | 3 |
| Ptn 7.2A4/4 | $10^4$ | 33.5 ± 2.1 | 1.0 ± 0.8 | 0 |
| Ptn 7.2A4/4 | $10^5$ | 33.4 ± 2.9 | 1.4 ± 0.9 | 0 |
| Ptn 7.2A4/4 | $10^6$ | 22.5 ± 4.1 | 3.4 ± 1.0 | 1 |

\* 7 birds per dose level/sporozoites given intracloacally.
\** Hematocrits were determined only on surviving birds.

The results in a second experiment showed that birds receiving E. necatrix sporozoites treated with Ptn7.2A4/4 monoclonal antibody were afforded some protection against infection. The lesion scores for groups receiving Ptn 7.2A4/4 were lower than respective controls groups.

| IN VIVO NEUTRALIZATION OF E. NECATRIX SPOROZOITES | | |
|---|---|---|
| Sporozoite Treatment | Sporozoite Dose* | Lesion Score $\bar{x}$ ± s.d. |
| Control | $10^4$ | 1.6 ± 0.5 |
| Control | $10^5$ | 1.8 ± 0.7 |
| Control | $10^6$ | 3.0 ± 0.9 |
| Ptn 7.2A4/4 | $10^4$ | 0.8 ± 0.4 |
| Ptn 7.2A4/4 | $10^5$ | 1.2 ± 0.4 |
| Ptn 7.2A4/4 | $10^6$ | 2.2 ± 0.7 |

\* 7 birds per dose level/sporozoites given orally.

## EXAMPLE 9

## USE OF E. TENELLA A4 ANTIGEN AND AN 11,500 DALTON FRAGMENT THEREOF TO ELICIT SPOROZOITE NEUTRALIZING SERUM RESPONSE AND PROTECTIVE RESPONSE AGAINST E. TENELLA IN CHICKENS

Eliciting Sporozoite Neutralizing Serum Response Against E. tenella Using the A4 Antigen.

The A4 antigen used in these experiments was prepared from sporocycts by methods described in Example 4 for the preparation of the nonreduced intact A4 antigen. Purity and identity of the protein was confirmed by SDS-PAGE and immunoreactivity with monoclonal antibody Ptn 7.2A4/4 prior to use in chickens.

Vaccine preparations were formulated at a level of one volume antigen to three volumes of oil carrier consisting of 5% Arlacel A, 94% Drakeol 6-VR, 1% Tween 80 so that each 0.1 ml dose contained approximately 15 $\mu$g of A4 antigen. When necessary, antigen was diluted with PBS (pH 7.2) to the level desired for formulation. Chickens received 0.1 ml dose by intramuscular route in the neck muscle. Antigen was administered two more times by the same route using the same amount at two-week intervals.

Three days prior to each administration of protein, and eleven days after the final administration, birds were bled for collection of serum samples. Heat inactivated sera were tested independently in the sporozoite microneutralization assay as described in Example 1.

The results as set forth below indicate that whereas non-vaccinated birds receiving carrier only had no demonstrable neutralizing antiserum titers against E. tenella sporozoites, birds receiving three doses of the antigen had demonstrable neutralizing antiserum titers.

| A4 Antigen Induced Sporozoite Neutralization Assay Data | | | |
|---|---|---|---|
| Serum Sample | Sporozoite Neutralization Titers (NDS) | | |
| | Highest | Lowest | Median Titer |
| Prebleed[a] | N.D.[b] | N.D. | N.D. |
| Nonvaccinate Controls (n = 9) | N.D. | N.D. | N.D. |
| Carrier Only (n = 14) | N.D. | N.D. | N.D. |
| Carrier/Protein Vaccine (n = 15) | 1:32 | N.D. | 1:8 |
| Immune serum[c] (Whole Sporozoite Vaccinates) | -- | -- | 1:32 |

[a] Serums from birds within each treatment group were pooled and tested.
[b] N.D. = No detectable neutralization.
[c] Pooled serum from several birds.

Eliciting a Protective Response in Chickens Using the A4 Antigen.

Sixty-three (63) days after the final vaccinataion, some birds were challenged, orally with 1,000 sporulated E. tenella oocysts. This was followed the next day with 3,000 sporulated E. tenella oocysts also given orally. Caecal lesions were scored 5 days after the final challenge. The results are tabulated below.

| Protection of A4 Antigen Vaccinate Birds Against E. tenella Coccidiosis | |
|---|---|
| | Lesion Score X̄±s.d. |
| Nonvaccinate Controls (n = 17) | 3.4 ± 0.6 |
| Adjuvant Only (n = 5) | 4.0 ± 0.0 |
| A4 Antigen/Adjuvant Vaccinates (n = 8) | 2.4 ± 1.3 |

Eliciting sporozoite neutralizing serum response against E. tenella using the 11,500 dalton fragment of the A4 antigen.

The 11,500 dalton immunogen used in these experiments was prepared from sporocysts by phenol extraction as described in Example 4. Purity and identity of the protein was confirmed by SDS-PAGE and immunoreactivity with monoclonal antibody Ptn 7.2A4/4 prior to use in chickens.

Lyophilized purified antigen was dissolved in 0.15 M phosphate buffered saline and emulsified in three parts carrier consisting of 5% Arlacel A, 94% Drakeol 6-VR, 1% Tween-80 at a final antigen concentration of 70 μg/ml. Chickens received 14 μg protein/0.2 cc dose by intra-muscular route in the neck muscle. Antigen was again administered two weeks later by the same route using the same amount.

One day prior to each administration of protein, and two weeks after the second administration of protein, birds were bled for collection of serum samples. Heat inactivated sera were tested independently in the sporozoite microneutralization assay as described in Example 1.

The results as set forth below indicate that whereas non-vaccinated birds receiving carrier only had no demonstrable neutralizing antiserum titers against E. tenella sporozoites, birds receiving two doses of antigen had demonstrable neutralizing antiserum titers of up to 1:81.

| Sporozoite Neutralization Assay Data | | | | |
|---|---|---|---|---|
| Serum Sample* | Sporozoite Neutralization Titers (NDS) | | | |
| | Bleeding | Highest | Lowest | Median Titers |
| Prebleed | 0 week | <1:3 | <1:3 | <1:3 |
| Non-vaccinate Controls | 2 weeks | <1:3 | <1:3 | <1:3 |
| | 4 weeks | <1:3 | <1:3 | <1:3 |
| Carrier only | 2 weeks | <1:3 | <1:3 | <1:3 |
| | 4 weeks | <1:3 | <1:3 | <1:3 |
| Carrier/protein vaccine | 2 weeks | <1:3 | <1:3 | <1:3 |
| | 4 weeks | 1:81 | <1:3 | 1:9 |
| Immune serum** (Whole Sporozoite vaccinates) | --- | --- | --- | 1:81 |

* 5 birds per group.
** Pooled serum from several birds.

Eliciting a protective response in chickens using the 11,500 dalton fragment of the A4 antigen.

Birds received approximately 3 μg of antigen in the aforementioned carrier one time in the neck muscle. A second group of birds received the carrier substance only. A final group of non-vaccinate [sentinal] birds was housed with each of the two aforementioned groups. Birds were exposed to coccidia by being housed in E. tenella contaminated cages. Approximately two weeks later, the birds were examined and found to have been infected by E. tenella. The following observations were noted.

| Protection of Vaccinate Birds Against Coccidiosis by E. tenella | | |
|---|---|---|
| Treatment | Lesion Score $\overline{X}$ ± s.d. | No. of Deaths |
| Adjuvant only (n = 5) | 3.8 ± 0.4 | 2 |
| Antigen vaccination (n = 5) | 1.0 ± 0.8 | 0 |
| Sentinal Birds (n = 6) | 4.0 ± 0.0 | 6 |

Because the conditions described above closely mimic the natural means of exposure to E. tenella in the field, the data presented show clear evidence of the usefulness of the invention for protection against coccidiosis due to E. tenella.

Demonstration that neutralizing serum antibodies of chickens recognize the 17,000 dalton polypeptide component of the A4 antigen.

Analysis of serum antibody specificity for the 17,000 dalton polypeptide component of the A4 antigen was performed using Western blots (4,46).All chicken sera with demonstrable neutralization titers to E. tenella sporozoites were shown to possess immunoglobulins with specificity for the 17,000 dalton polypeptide component of the A4 antigen; conversely, no sera from non-responding or control birds had specificity for the 17,000 dalton polypeptide or any other sporozoite protein.

Demonstration that neutralization serum antibodies of chickens compete with monoclonal antibody Ptn 7.2A4/4.

Sera from vaccinated birds with demonstrable neutralization titers to E. tenella sporozoites, as well as corresponding control sera were tested for the ability to compete with antibody Ptn 7.2A4/4 for binding sites on sporozoite membranes. Polystyrene 96 well clusters (Immulon II) were sensitized with 50 μl of sporozoite membrane proteins in 10 mM glycine buffered saline, pH 9.6, at a level of approximately 100 μg total protein/ml. Serial two-fold dilutions of sera were prepared in 0.15M phosphate buffered saline with 0.0005% Tween-20 containing a 1:80 dilution of alkaline phosphatase conjugated to Ptn 7.2A4/4 and then transferred to the sensitized plates at a final volume of 75 μl/well. After incubation at 37ºC for 30 minutes, the plates were rinsed free of unreacted materials using 0.15M phosphate buffered saline with (0.0005%) Tween-20. Afterward, substrate consisting of the sodium salt of phosphonitrophenol dissolved in 100 mM diethanoline buffer at a level of 1 mg/ml was added to each well of the plate to a final volume of 100 μ l. The resultant reaction product was monitored spectrophometrically. From the study it was possible to ascertain that sera from birds responding to the vaccination as evidenced by neutralization and immunoblots also contained antibody which competed with monoclonal antibody Ptn 7.2A4/4. This experiment provides direct evidence that antigen purified from sporozoite membranes by either immunoaffinity chromatography using monoclonal Ptn 7.2A4/4 or conventional chromatography is capable of stimulating an immune response in chickens to the epitope defined by monoclonal Ptn 7.2A4/4.

EXAMPLE 10


USE OF E. TENELLA PROTEIN TO ELICIT
SPOROZOITE NEUTRALIZING SERUM RESPONSE
AGAINST E. NECATRIX IN CHICKENS


Heat inactivated sera from birds vaccinated with the 11,500 dalton fragment of the A4 antigen (Example 9) were pooled and tested in the neutralization assay (Example 1) substituting embryonic porcine lung cells for embryonic chick kidney cells. The results were as listed in the following table.

| Treatment | Neutralization Titer |
|---|---|
| Non-immune chicken serum | <1:12 |
| A4 Antigen Vaccination | 1:48 |
| E. tenella whole sporozoite immune serum | 1:48 |

The data demonstrate the development of an elevated serum neutralization titer against E. necatrix when birds receive the purified 11,500 dalton fragment of the A4 antigen. Since it was previously demonstrated that administration of the A4 antigen or the 11,500 dalton fragment of the A4 antigen results in the elevation of serum neutralizing titers to E. tenella, and that administration of the A4 antigen or the 11,500 dalton fragment of the A4 antigen results in protection from E. tenella challenge, and since E. necatrix sporozoite neutralization titers are elevated by the administration of the 11,500 dalton fragment of the A4 antigen, one may infer that protection against E. necatrix challenge will also result from administration of either the A4 antigen or the 11,500 dalton fragment of the A4 antigen.

EXAMPLE 11

## FORMULATION AND USE OF ANTIGEN FOR PROTECTION OF CHICKENS AGAINST DISEASE CAUSED BY E. TENELLA

A composition for immunization of chickens against coccidiosis caused by E. tenella may be prepared from the intact A4 antigen identified by monoclonal antibody Ptn 7.2A4/4 or a fragment thereof. One suitable carrier for the antigen is 5% Arlacel A, 94% Drakeol 6-VR, 1% Tween-80. The vaccine may be prepared by formulating one part of an aqueous solution of the antigen with 3 parts Arlacel A/Drakeol 6-VR to a final concentration of 10 $\mu$g antigen/dose. The vaccine may be administered to chickens of any age by the intramuscular route. Properly vaccinated birds would be protected against disease, depressed performance or death caused by field challenge with E. tenella.

### EXAMPLE 12

## FORMULATION AND USE OF ANTIGEN FOR PROTECTION OF CHICKENS AGAINST DISEASE CAUSED BY E. NECATRIX

One composition may include that described in Example 10. The vaccine may be administered to chickens of any age by the intramuscular route. Properly vaccinated birds would be protected against disease, depressed performance or death caused by field challenge with E. necatrix.

### References

1. Ali, N.S., Binnerts, W.T. and Klimes, B. (1972). Immunization by (sic) irradiated Eimeria acervulina. J. Prot. 19, 177.

2. Benton, W.D. and Davis, R.W. (1977). Screening λgt recombinant clones by hybridization to single plaques in situ. Science 196, 180-182.

3. Blobel, G. and Dobberstein, B. (1975). Transfer of proteins across membranes I. Presence of proteolytically processed and unprocessed nascent immunoglobulin light chains on membrane-bound ribosomes of murine myeloma. J. Cell Biol. 67, 835-851.

4. Burnette, W.M. (1981). "Western Blotting": electrophoretic transfer of proteins from sodium dodecyl sulfate - polyacrylamide gels to unmodified nitrocellulose and radiographic detection with antibody and radioiodinated protein A. Anal. Biochem. 112, 195.

5. Cohen, S.A., Tarvin, T.L. and Bidlingmeyer, B.A. (1984). Analysis of amino acids using precolumn derivatization with phenylisothiocyanate. American Laboratory 16, 48-59.

6. Danforth, H.D. (1982). Development of hybridoma-produced antibodies directed against Eimeria tenella and E. mitis. J. Parasitol. 68, 392.

7. Danforth, H.D. (1983). Use of monoclonal antibodies directed against Eimeria tenella sporozoites to determine stage specificity and in vitro effect on parasite penetration and development. Am J. Vet. Res 44, 1722.

8. Danforth, H.D. and Augustine P.C. (1983). Specificity and cross-reactivity of immune serum and hybridoma antibodies to various species of avian coccidia. Poultry Science 62, 2145.

9. Davis, P.J., Parry, S.H. and Porter, P. (1978). The role of secretory IgA in anti-coccidial immunity in the chicken. Immunology 34, 879.

10. Davis, P.J. and Porter, P. (1979). A mechanism for secretory IgA mediated inhibition of the cell penetration and intracellular development of Eimeria tenella. Immunology 36, 471.

11. Dvorak, J.A. and Crane, M. St. J. (1981). Vertebrate cell cycle modulates infection by protozoan parasites. Science 214, 1034.

12. Edman, P. and Begg, G. (1967). A protein sequenator. Automated Equipment for Sequence determination, Eur. J. Biochem 1, 80.

13. Giambrone, J.J., Klesius, P.H. and Edgar, S.A. (1980). Avian Coccidiosis: Evidence for a cell-mediated immune response. Poultry Sci. 59, 38.

14. Gibbons, R.A., Sellwood, R., Burrow, M. and Hunter, P.A. (1977). Inheritance of resistance to neonatal E. coli diarrhea in the pig: examination of the genetic system. Theor. Appl. Genet. 51, 65.

15. Gore, T.C., Long, P.L., Kogut, M. and Johnson, J. (1983). Attenuation of Eimeria necatrix and E. tenella of U.S. origin by serial embryo passage. Avian Disease 27, 569.

16. Hunkapiller, M.W., Hewick, R.M., Dreyer, W.J. and Hood, L.E. (1983). High sensitivity sequencing with a gas phase sequenator. Methods in Enzymology 91, Academic Press, New York, 399-413.

17. Hunkapiller, M.W., Strickler, J.E. and Wilson, K.J. (1984). Contemporary Methodology for Protein Structure Determination. Science 226, 304-311.

18. Jeffers, T.K. (1975). Attenuation of Eimeria tenella through selection for precociousness. J. Parasitol. 61, 1083.

19. Jeffers, T.K. (1976). Genetic recombination of precociousness and anticoccidial drug resistance on Eimeria tenella. Zeitschrift fur Parasitenkunde 50, 251.

20. Johnson, J. and Reid, W.M. (1970). Anticoccidial Drugs: Lesion scoring techniques in battery and floor pen experiments with chickens. Exp. Parasitology 38, 36.

21. Kasper, L.H., Crabb, J.H., and Pfefferkorn, E.R. (1983). Purification of a major membrane protein of Toxoplasma gondii by immunoabsorption with a monoclonal antibody. J. Immunol. 130, 2407.

22. Keusch, G.T. (1979). Specific membrane receptors: Pathogenic and therapeutic implications in infectious diseases. Rev. Inf. Dis. 1, 517.

23. Kriel, G. (1981). Transport of proteins across membranes. Ann. Rev. Biochem. 50, 317-348.

24. Laemmli, U.K. (1970). Cleavage of structural proteins during the assembly of the head of bacteriophage T4, Nature 227, 680.

25. Leder, P., Tiemeier, D. and Enquist, L. (1977). EK2 derivatives of bacteriophage lambda useful in cloning of DNA from higher organisms: the λ gtWES system. Science 196, 175-177.

26. Long, P.L. (1972) Eimeria mivati: Reproduction, pathogenicity and immunogenicity of a strain maintained in chick embryos by serial passage. J. Comp. Pathol. 82, 839.

27. Long, P.L. (1974). Further studies on the pathogenicity and immunogenicity of an embryo adapted strain of Eimeria tenella. Avian Pathology 3, 255.

28. Long, P.L. (1982). The Biology of the Coccidia. University Park Press, Baltimore. Pg. 44.

29. Long, P.L., Johnson, J., and Gore, T.C. (1982). Attenuation of a strain of Eimeria mivati of U.S. origin by serial embryo passage. Avian Diseases. 26, 305.

30. Long, P.L. and Rose, M.E. (1965). Active and passive immunization of chickens against induced infections of Eimeria tenella. Exp. Parasit. 16, 1.

31. Lowder, L.J. (1966). Artificial acquired immunity to Eimeria bovis infections in cattle. Proc. Int. Congr. Parasit. 1, 106.

32. Maniatis, T., Fritsch, E.F. and Sambrook, J. (1982). Molecular Cloning - A Laboratory Manual, Cold Springs Harbor Laboratory, New York.

33. Marquardt, W.C. (1980). Host and site specificity in the coccidia; a perspective. J. Protozool. 26, 243.

34. Maxam, A. and Gilbert, W. (1980). Sequencing end-labelled DNA with base-specific chemical change in: Methods in Enzymology, Vol. 65, part 1, Academic Press, New York, pp. 499-559.

35. McDonald, V. and Ballingall, S. (1983). Attenuation of Eimeria mivati (: mitis) by selection for precocious development. Parasitology 86, 371.

36. McDonald, V. and Ballingall, S. (1982). Further investigation of the pathogenicity, immunogenicity and stability of precocious Eimeria acervulina. Parasitology 86, 361.

37. McDonald, V., Ballingall, S. and Shirley, M.W. (1982). A preliminary study of the nature of infection and immunity in chickens given an attenuated line of Eimeria acervulina. Parasitology 84, 21.

38. McDougald, L.R. Status of coccidiosis: new products on way. Poult. Digest. Oct., 1981.

39. McDougald, L.R. New anticoccidial drugs: Better things to come or "endangered species?" Feedstuffs Aug. 15, 1983.

40. Miller, L.H., Mason, S.J., Dvorak, J.A., McGinniss, M.H., and Rothman, I.K. (1975) Erythrocyte receptors of (Plasmodium knowlesi) Malaria: Duffy blood group determinants. Science 189, 561.

41. Reid, W.M. (1978). Protozoa. In: Diseases of Poultry. 7th ed. M.S. Hofstad, ed. Iowa State Univ. Press. pp. 942-1054.

42. Riley, J.F. (1980). Screening for and evaluation of anticoccidial activity. Adv. Pharm. Chemo. 17, 1.

43. Rose, M.E. (1974). Immune responses to the Eimeria: Recent observations. Sympo. Coccidia and Related Organisms. pp. 92-118. Univ. Guelph, Ontario.

44. Sanger, F. and Coulson, A.R. (1978). The use of thin polyacrylamide gels for DNA sequencing. FEBS Lett. 87, 107-110.

45. Schmidt, G.O. and Roberts, L.S. (1977). Foundations of Parasitology. Mosby Co., St. Louis. pp. 122-128.

46. Sharma, S.D., Mullenax, J., Araujo, F.G., Erlich, H.A. and Remington, J.S. (1983). Western blot analysis of the antigens of Toxoplasma gondii recognized by human IgM and IgG antibodies. J. Immunology 131, 977.

47. Sharp, P.A. (1981). Speculations on RNA splicing. Cell 23, 643-646.

48. Shirley, M.W. (1980) Eimeria necatrix: The development and characteristics of an egg-adapted (attenuated) line. Parasitology 81, 525.

49. Shirley, M.W. (1982). Features of an attenuated line of Eimeria praecox. Parasitology. Proceedings of the British Soc. for Parasitology 81, 525.

50. Shirley, M.W., Bellatti, M.A. and Millard, B.J. (1982). An egg-shaped (attenuated) line of Eimeria necatrix: further studies on its reproduction pathogenicity and immunogenicity. Parasitology 84, 215.

51. Speer, D.A., Wong, R.B. and Schenkel, R.H. (1983). Effects of monoclonal IgG antibodies on Eimeria tenella (coccidia) sporozoites. J. Parasitol. 69, 775.

52. Speer, C.A., Wong, R.B. and Schenkel, R.H. (1983). Ultrastructural localization of monoclonal IgG antibodies for antigenic sites of Eimeria tenella oocysts, sporocysts and sporozoites. J. Protozoal. 30, 548.

53. Steiner, D.F., Quinn, P.S., Chan, S.J., Marsh, J. and Tager, H.S. (1980). Processing mechanisms in the biosynthesis of proteins. Annals N.Y. Acad. Sci. 343, 1-16.

54. Svennerholm, A., Lange, S. and Holmgrin, J. (1978). Correlation between intestinal synthesis of specific immunoglobulin A and protection against experimental cholera in mice. Inf. Imm. 21, 1.

55. Van Deusen, R.A. and Whetstone, C.A. (1981). Practical aspects of producing anti-viral monoclonal antibodies as diagnostic reagents. Proc. Amer. Assn. Vet. Lab. Diagnost. 24, 211.

56. Viera, J. and Messing, J. (1982). The pUC plasmids, an M13mp7-derived system for insertion mutagenesis and sequencing with synthetic universal primers. Gene 19 259-268.

57. Wisher, M.H. (1983). J. Cellular Biochem., Supp. 7A, Abstract 0059.

58. Wong, R.B. and Schenkel, R.H. (1984). Fed. Proc. 184, 43(6), 1630.

59. Wright, I.G., White, M., Tracey-Patte, P.D., Donaldson, R.A., Goodger, B.V., Waltisbuhl, O.J. and Mahoney, D.F. (1983). Babesia bovis: Isolation of a protective antigen by using monoclonal antibody. Infection and Immunity 41, 244.

60. Wrigley, C.W. (1971). Gel Electrofocusing in: Methods in Enzymology Vol. XXII, W.B. Jacoby ed., Academic Press. pp. 559-564.

**Claims**

1. A purified antigenic protein capable of inducing in a chicken an immune response conferring protection against infection by Eimeria tenella, the protein having a molecular weight of about 25,000 and being composed of two polypeptides joined by a disulfide bond, one of the polypeptides being characterized by a molecular weight of about 17,000 and by a blocked N-terminal amino acid and having the amino acid sequence set forth in Figure (3) and the other of the polypeptides being characterized by a molecular weight of about 8,000 and having the amino acid sequence set forth in Figure (3).

2. A method of preparing the protein of claim 1 which comprises :
   a) contacting sporocysts of Eimeria tenella with a detergent under suitable non-reducing conditions in the presence of protease inhibitors so as to solubilize the sporocyst membrane proteins ; and
   b) separately recovering the protein from the solubilized, sporocyst membrane proteins under suitable non-reducing conditions, the separately recovering comprising immunoprecipitation or immunoaffinity chromatography with monoclonal antibody Ptn 7.2 A4/4 produced by hybridoma cell line ATCC No. HB 8561.

3. A method of preparing the 17,000 dalton polypeptide of claim 1 which comprises :
   a) contacting sporocysts of Eimeria tenella with a detergent under suitable conditions in the presence of protease inhibitors so as to solubilize the sporocyst membrane proteins ; and
   b) separately recovering the polypeptide from the solubilized, sporocyst membrane proteins under suitable reducing conditions, the separately recovering comprising partially purifying the solubilized sporocyst membrane proteins by chromatography on DEAE-cellulose followed by preparative SDS-electrophoresis under suitable reducing conditions.

4. A method of preparing an antigenic polypeptide having an amino acid sequence included within the amino acid sequence of the polypeptide of claim 1 and characterized by a molecular weight of about 11,500, which comprises :
   a) contacting sporocysts of Eimeria tenella with phenol in the presence of 8-hydroxyquinoline so as to extract the sporozoite membrane proteins from the sporocysts ; and
   b) recovering the polypeptide from the extracted sporocyst membrane proteins by immunoprecipitation or immunoaffinity chromatography with monoclonal antibody Ptn 7.2 A4/4 produced by hybridome cell line ATCC No. HB 8561.

EP 0 164 176 B1

**5.** A method of preparing an antigenic polypeptide having an amino acid sequence included within the amino acid sequence of the polypeptide of claim 1 and characterized by a molecular weight of about 6,500, which comprises :

a) contacting trypsin-taurodeoxycholate excysted, sporozoite membrane proteins with detergent so as to solubilize the proteins ; and

b) recovering the polypeptide from the solubilized, excysted sporozoite membrane proteins by immunoprecipitation or immunoaffinity chromatography with monoclonal antibody Ptn 7.2 A4/A produced by hydriboma line ATCC No. HB 8561.

**6.** A method of preparing the protein of claim 1 which comprises preparing a DNA molecule coding for the protein, inserting the DNA molecule into an appropriate expression vector, introducing the resulting expression vector into a suitable host under appropriate conditions permitting expression of the DNA and production of the protein and recovering the protein so produced.

**7.** A method of preparing an antigenic polypeptide having an amino acid sequence included within the amino acid sequence of the protein of claim 1 and capable of inducing in a chicken an immune response conferring protection against infection by Eimeria tenella, which comprises preparing a DNA molecule coding for the polypeptide, inserting the DNA molecule into an appropriate expression vector, introducing the resulting expression vector into a suitable host under appropriate conditions permitting expression of the DNA and production of the polypeptide and recovering the polypeptide so produced.

**8.** A method of preparing the 17,000 dalton polypeptide of claim 1 which comprises preparing a DNA molecule coding for the polypeptide, inserting the DNA molecule into an appropriate expression vector, introducing the resulting expression vector into a suitable host under appropriate conditions permitting expression of the DNA and production of the polypeptide and recovering the polypeptide so produced.

**9.** A vaccine for conferring upon a chicken active immunity against infection by Eimeria tenella which comprises per dose an effective immunizing amount of the protein of claim 1 and suitable carrier.

**10.** A vaccine for conferring upon a chicken active immunity against infection by Eimeria tenella which comprises an effective immunizing amount of the polypeptide of claim 7 and a suitable carrier.

**11.** A vaccine of claim 9, wherein the effective immunizing amount is above about 0.1 $\mu$g/kg of body weight of the chicken.

**12.** A monoclonal antibody directed against the protein of claim 1.

**13.** A monoclonal antibody directed against the polypeptide of claim 7.

**14.** The monoclonal antibody Ptn 7.2A4/4 produced by hybridoma cell line ATCC No. HB8561.

**15.** A composition for conferring upon a chicken passive immunity against infection by Eimeria tenella which comprises an effective protecting amount of the monoclonal antibody of claim 12, 13 or 14 and a suitable carrier.

**16.** An anti-idiotypic antibody directed against the monoclonal antibody of claim 14.

**17.** A method of preparing the anti-idiotypic antibody of claim 16 which comprises :

a) recovering the monoclonal antibody Ptn 7.2A4/4 from hybridoma cell line ATCC No. HB8561 ;

b) purifying the monoclonal antibody ;

c) injecting the purified monoclonal antibody into a suitable animal together with a suitable adjuvant ;

d) removing blood serum from the injected animal ; and

e) recovering the anti-idiotypic antibody from the blood serum.

**18.** A nucleic acid molecule encoding the protein of claim 1.

**19.** A DNA molecule of claim 18 having the nucleic acid sequence set forth in Figure (3).

27

EP 0 164 176 B1

**20.** A cDNA molecule of claim 18.

**21.** A DNA molecule encoding the polypeptide of any of claims 4, 5 or 7.

**22.** A cloning vehicle which comprises the nucleic acid of claim 18.

**23.** A host cell which comprises the cloning vehicle of claim 22.

**24.** A bacterial host of claim 23.

**25.** A method of producing the protein having the amino acid sequence set forth in Figure (3) which comprises growing host cells of claim 23 under suitable conditions permitting production of the protein and recovering the protein so produced.

**26.** A method for obtaining the DNA of claim 19 which comprises isolating total genomic DNA from Eimeria tenella oocysts ; preparing DNA fragments from the genomic DNA so isolated ; ligating the fragments so prepared into an appropriate cloning vector ; subjecting the DNA of the clones so prepared to hybridization with oligonucleotides containing, or complementary to, nucleic acid sequences present within the nucleic acid sequence set forth in Figure (3) to identify appropriate clones ; and isolating from the appropriate clones DNA which encodes the protein and has the nucleic acid sequence set forth in Figure (3).

**Patentansprüche**

**1.** Gereinigtes antigenisches Protein, das in eine Huhn eine Immunresponse verursachen kann, die Schutz gegen Infektion durch Eimeria tenella gewährt, welches Protein ein Molekulargewicht von ca. 25.000 aufweist und aus zwei Polypeptiden zusammengesetzt ist, die durch eine Disulfidbindung vereinigt sind, wobei eines der Polypeptide durch ein Molekulargewicht von ca. 17.000 und durch eine blockierte N-endständige Aminosäure gekennzeichnet ist und die in Figure (3) gezeigte Aminosäuresequenz aufweist, und wobei das andere der Polypeptide durch ein Molekulargewicht von ca. 8.000 gekennzeichnet ist und die in Figure (3) gezeigte Aminosäuresequenz aufweist.

**2.** Verfahren zur Herstellung des Proteins nach Anspruch 1, wobei man:
a) Sporozysten von Eimeria tenella unter geeigneten nichtreduzierenden Bedingungen in Gegenwart von Proteaseinhibitoren mit einem Detergens in Berührung bringt, um die Sporozystenmembranproteine zu solubilisieren; und
b) das Protein unter geeigneten nicht-reduzierenden Bedingungen separat aus den solubilisierten Sporozystmembranproteinen zurückgewinnt, wobei die separate Rückgewinnung Immunpräzipitation oder Immunaffinitätschromatographie mit dem durch die Hybridomazellinie ATCC Nr. HB 8561 erzeugten monoklonalen Antikörper Ptn 7.2 A4/4 umfaßt.

**3.** Verfahren zur Herstellung von 17.000 Dalton Polypeptid nach Anspruch 1, wobei man:
a) Sporozysten von Eimeria tenella unter geeigneten Bedingungen in Gegenwart von Proteaseinhibitoren mit einem Detergens in Berührung bringt, um die Sporozystmembranproteine zu solubilisieren; und
b) das Polypeptid unter geeigneten reduzierenden Bedingungen separat aus den solubilisierten Sporozystmembranproteinen zurückgewinnt, wobei die separate Rückgewinnung teilweise Reinigung der solubilisierten Sporozystmembranproteine durch Chromatographie auf DEAE-Cellulose mit anschließender präparativer SDS-Elektrophorese unter geeigneten reduzierenden Bedingungen umfaßt.

**4.** Verfahren zur Herstellung eines antigenischen Polypeptids, das eine innerhalb der Aminosäuresequenz des Polypeptids nach Anspruch 1 befindliche Aminosäuresequenz aufweist und durch ein Molekulargewicht von ca. 11.500 gekennzeichnet ist, wobei man:
a) Sporozysten von Eimeria tenella in Gegenwart von 8-Hydroxychinolin mit Phenol in Berührung bringt, um die Sporozoitmembranproteine aus den Sporozysten zu extrahieren; und
b) das Polypeptid durch Immunpräzipitation oder Immunaffinitätschromatographie mit dem durch die Hybridomazellinie ATCC Nr. HB 8561 erzeugten monoklonalen Antikörper Ptn 7.2 A4/4 aus den extrahierten Sporozystmembranproteinen zurückgewinnt.

28

**5.** Verfahren zur Herstellung eines antigenischen Polypeptids, das eine innerhalb der Aminosäuresequenz des Polypeptids nach Anspruch 1 befindliche Aminosäuresequenz aufweist und durch ein Molekulargewicht von ca. 6.500 gekennzeichnet ist, wobei man:
a) Trypsin-Taurodesoxycholate-exzystierte Sporozoitmembranproteine mit Detergens in Berührung bringt, um die Proteine zu solubilisieren; und
b) das Polypeptid durch Immunpräzipitation oder Immunaffinitätschromatographie mit dem durch die Hybridomazellinie ATCC Nr. HB 8561 erzeugten monoklonalen Antikörper Ptn 7.2 A4/4 aus den solubilisierten, exzystierten Sporozystenmembranproteinen zurückgewinnt.

**6.** Verfahren zur Herstellung des Proteins nach Anspruch 1, wobei man ein für das Protein kodierendes DNA-Molekül herstellt, das DNA-Molekül in einen geeigneten Expressionsvektor einfügt, den resultierenden Expressionsvektor unter geeigneten Bedingungen, die Expression der DNA und Erzeugung des Proteins und Rückgewinnung des so hergestellten Proteins gestatten, in einen geeigneten Wirt einführt.

**7.** Verfahren zur Herstellung eines antigenischen Polypeptids, das eine innerhalb der Aminosäuresequenz des Polypeptids nach Anspruch 1 befindliche Aminosäuresequenz aufweist und in einem Huhn eine Immunresponse verursachen kann, die Schutz gegen Infektion durch Eimeria tenella gewährt, wobei man ein für das Polypeptid kodierendes DNA-Molekül herstellt, das DNA-Molekül in einen geeigneten Expressionsvektor einfügt, den resultierenden Expressionsvektor unter geeigneten Bedingungen, die Expression der DNA und Erzeugung des Polypeptids und Rückgewinnung des so hergestellten Polypeptids gestatten, in einen geeigneten Wirt einführt.

**8.** Verfahren zur Herstellung von 17.000 Dalton Polypeptid nach Anspruch 1, wobei man ein für das Polypeptid kodierendes DNA-Molekül herstellt, das DNA-Molekül in einen geeigneten Expressionsvektor einfügt, den resultierenden Expressionsvektor unter geeigneten Bedingungen, die Expression der DNA und Erzeugung des Polypeptids und Rückgewinnung des so erzeugten Polypeptids gestatten, in einen geeigneten Wirt einführt.

**9.** Vakzin zur Herbeiführung in einem Huhn einer wirksamen Immunität gegen Infektion durch Eimeria tenella, welches Vakzin je Dosis eine wirksame immunisierende Menge des Proteins nach Anspruch 1 und einen geeigneten Träger enthält.

**10.** Vakzin zur Herbeiführung in einem Huhn einer wirksamen Immunität gegen Infektion durch Eimeria tenella, welches Vakzin eine wirksame immunisierende Menge des Polypeptids nach Anspruch 7 und einen geeigneten Träger enthält.

**11.** Vakzin nach Anspruch 9, bei dem die wirksame immunisierende Menge über ca. 0,1 $\mu$g/kg des Körpergewichts des Huhns liegt.

**12.** Monoklonaler Antikörper, gerichtet gegen das Protein nach Anspruch 1.

**13.** Monoklonaler Antikörper, gerichtet gegen das Polypeptid nach Anspruch 7.

**14.** Monoklonaler Antikörper Ptn 7.2 A4/4, erzeugt durch die Hybridomazellinie ATCC Nr. HB 8561.

**15.** Zusammensetzung zur Herbeiführung in einem Huhn einer passiven Immunität gegen Infektion durch Eimeria tenella, welche Zusammensetzung eine wirksame Schutzmenge des monoklonalen Antikörpers nach Anspruch 12, 13 oder 14 und einen geeigneten Träger enthält.

**16.** Anti-idiotypischer Antikörper, gerichtet gegen den monoklonalen Antikörper nach Anspruch 14.

**17.** Verfahren zur Herstellung des anti-idiotypischen Antikörpers nach Anspruch 16, wobei man:
a) den monoklonalen Antikörper Ptn 7.2 A4/4 aus der Hybridomazellinie ATCC Nr. HB 8561 zurückgewinnt;
b) den monoklonalen Antikörper reinigt;
c) den gereinigten monoklonalen Antikörper in ein geeignetes Tier injiziert, zusammen mit einem geeigneten Hilfsmittel;
d) Blutserum aus dem injizierten Tier entfernt; und

e) den anti-idiotypischen Antikörper aus dem Blutserum zurückgewinnt.

**18.** Nukleinsäuremolekül, welches das Protein nach Anspruch 1 enkodiert.

**19.** DNA-Molekül nach Anspruch 18 mit der in Figur (3) gezeigten Nukleinsäuresequenz.

**20.** cDNA-Molekül nach Anspruch 18.

**21.** DNA-Molekül, welches das Polypeptid nach einem der Ansprüche 4, 5 oder 7 enkodiert.

**22.** Klonierender Träger, der die Nukleinsäure nach Anspruch 18 enthält.

**23.** Wirtzelle, die den klonierenden Träger nach Anspruch 22 enthält.

**24.** Bakterieller Wirt nach Anspruch 23.

**25.** Verfahren zur Erzeugung des Proteins mit der in Figur (3) gezeigten Aminosäuresequenz, wobei man Wirtzellen nach Anspruch 23 unter geeigneten Bedingungen, die Erzeugung des Proteins und Rückgewinnung des so erzeugten Proteins gestatten, wachsen läßt.

**26.** Verfahren zur Erlangung der DNA nach Anspruch 19, wobei man totale genomische DNA aus Eimeria tenella-Oozysten isoliert; DNA-Fragmente aus der so isolierten genomischen DNA herstellt; die so hergestellten Fragmente in einen geeigneten klonierenden Vektor ligiert; die DNA der so hergestellten Klone einer Hybridisierung mit Oligonukleotiden unterwirft, enthaltend, oder komplementär mit, Nukleinsäuresequenzen, die innerhalb der in Figur (3) gezeigten Nukleinsäuresequenz anwesend sind, um geeignete Klone zu identifizieren; und aus den geeigneten Klonen DNA isoliert, die das Protein enkodiert und die in Figur (3) gezeigte Nukleinsäuresequenz aufweist.

**Revendications**

**1.** Une protéine antigénique purifiée, capable d'induire, chez un poulet, une réponse immune conférant une protection contre une infection par Eimeria tenella, la protéine ayant un poids moléculaire d'environ 25 000 et se composant de deux polypeptides unis par un lien disulfurique, l'un des polypeptides étant caractérisé par un poids moléculaire d'environ 17 000 et par un acide aminé N-terminal bloqué et ayant la séquence d'acides aminés illustrée à la Figure (3), l'autre polypeptide étant caractérisé par un poids moléculaire d'environ 8 000 et ayant la séquence d'acides aminés illustrée à la Figure (3).

**2.** Une méthode de préparation de la protéine de la revendication 1, qui comprend :
a) la mise en contact de sporocystes d'Eimeria tenella avec un détergent dans des conditions non réductrices correctes en présence d'inhibiteurs de protéase, afin de solubiliser les protéines membraneuses de sporocystes; et
b) la récupération séparée de la protéine à partir des protéines membraneuses solubilisées de sporocystes dans des conditions non-réductrices correctes, la récupération séparée comprenant l'immunoprécipitation ou la chromatographie par immuno-affinité avec l'anticorps monoclonal Ptn 7.2 A4/4 produit par la ligne cellulaire de l'hybridome ATCC n°. HB 8561.

**3.** Une méthode de préparation du polypeptide de 17 000 daltons de la revendication 1, qui comprend :
a) la mise en contact de sporocystes d'Eimeria tenella avec un détergent dans des conditions correctes, en présence d'inhibiteurs de protéase, afin de solubiliser les protéines membraneuses des sporocystes; et
b) la récupération séparée du polypeptide à partir des protéines membraneuses solubilisées des sporocystes dans des conditions réductrices correctes, la récupération séparée comprenant la purification partielle des protéines membraneuses solubilisées des sporocystes par chromatographie sur cellulose DEAE, suivie par une électrophorèse SDS préparatoire dans des conditions réductrices correctes.

**4.** Une méthode de préparation d'un polypeptide antigénique ayant une séquence d'acides aminés inclue dans la séquence d'acides aminés du polypeptide de la revendication 1 et caractérisé par un poids moléculaire d'environ 11 500, qui comprend :

    a) la mise en contact de sporocystes d'Eimeria tenella avec du phénol en présence de 8-hydroxyquinoline, afin d'extraire les protéines membraneuses de sporozoïtes des sporocystes; et

    b) la récupération du polypeptide à partir des protéines membraneuses de sporocystes extraites par immunoprécipitation ou chromatographie par immuno-affinité avec l'anticorps monoclonal Ptn 7.2A4/4 produit par ligne cellulaire de l'hybridome ATCC n°. HB 8561.

**5.** Une méthode de préparation d'un polypeptide antigénique ayant une séquence d'acides aminés inclue dans la séquence d'acides aminés du polypeptide de la revendication 1 et caractérisé par un poids moléculaire d'environ 6 500, qui comprend :

    a) la mise en contact de protéines membraneuses de sporozoïtes excystés par trypsine-taurodéoxycholate avec du détergent afin de solubiliser les protéines; et

    b) la récupération du polypeptide à partir des protéines membraneuses solubilisées de sporozoïtes excystés par immunoprécipitation ou par chromatographie par immuno-affinité avec l'anticorps monoclonal Ptn 7.2 A4/4 produit par ligne d'hybridome ATCC n°. HB 8561.

**6.** Une méthode de préparation de la protéine de la revendication 1 qui comprend la préparation d'une molécule d'ADN codant la protéine, l'insertion de la molécule d'ADN dans un vecteur d'expression approprié, l'introduction du vecteur d'expression en résultant dans un hôte adéquat dans des conditions appropriées, permettant l'expression de l'ADN et la production de la protéine, ainsi que la récupération de la protéine ainsi produite.

**7.** Une méthode de préparation d'un polypeptide antigénique ayant une séquence d'acides aminées inclue dans la séquence d'acides aminés de la protéine de la revendication 1 et capable d'induire dans un poulet une réponse immune conférant une protection contre l'infection par Eimeria tenella, qui comprend la préparation d'une molécule d'ADN codant le polypeptide, l'insertion de la molécule d'ADN dans un vecteur d'expression approprié, l'introduction du vecteur d'expression en résultant dans un hôte approprié dans des conditions correctes, permettant l'expression de l'ADN et la production du polypeptide, ainsi que la récupération du polypeptide ainsi produit.

**8.** Une méthode de préparation du polypeptide de 17 000 daltons de la revendication 1, qui comprend la préparation d'une molécule d'ADN codant le polypeptide, l'insertion de la molécule d'ADN dans un vecteur d'expression approprié, l'introduction du vecteur d'expression en résultant dans un hôte adéquat dans des conditions appropriées, permettant l'expression de l'ADN et la production du polypeptide, ainsi que la récupération du polypeptide ainsi produit.

**9.** Un vaccin conférant à un poulet une immunité active contre une infection par Eimeria tenella qui comprend, par dose, une quantité immunisante effective de la protéine de la revendication 1 et un support adéquat.

**10.** Un vaccin conférant à un poulet une immunité active contre une infection par Eimeria tenella, qui comprend une quantité immunisante effective du polypeptide de la revendication 7 et un support adéquat.

**11.** Un vaccin de la revendication 9, dans lequel la quantité immunisante effective est supérieure à environ 0,1 $\mu$g/kg de poids corporel du poulet.

**12.** Un anticorps monoclonal dirigé contre la protéine de la revendication 1.

**13.** Un anticorps monoclonal dirigé contre le polypeptide de la revendication 7.

**14.** L'anticorps monoclonal Ptn 7.2 A4/4 produit par la ligne cellulaire d'hybridome ATCC n°. HB8561.

**15.** Une composition conférant à un poulet une immunité passive contre une infection par Eimeria tenella, qui comprend une quantité protectrice effective de l'anticorps monoclonal des revendications 12, 13 ou 14 et un support adéquat.

**16.** Un anticorps anti-idiotypique dirigé contre l'anticorps monoclonal de la revendication 14.

**17.** Une méthode de préparation de l'anticorps anti-idiotypique de la revendication 16 qui comprend :

a) la récupération de l'anticorps monoclonal Ptn 7.2A4/4 de la ligne cellulaire d'hybridome ATCC n°. HB 8561;

b) la purification de l'anticorps monoclonal;

c) l'injection de l'anticorps monoclonal purifié à un animal adéquat, avec un adjuvant approprié;

d) l'extraction de sérum sanguin de l'animal injecté; et

e) la récupération de l'anticorps anti-idiotypique du sérum sanguin.

**18.** Une molécule d'acide nucléique codant la protéine de la revendication 1.

**19.** Une molécule d'ADN de la revendication 18 ayant la séquence d'acide nucléique illustrée à la Figure (3).

**20.** Une molécule d'ADNc de la revendication 18.

**21.** Une molécule d'ADN codant le polypeptide des revendications 4, 5 ou 7.

**22.** Un véhicule de clonage comprenant l'acide nucléique de la revendication 18.

**23.** Une cellule hôte comprenant le véhicule de clonage de la revendication 22.

**24.** Un hôte bactérien de la revendication 23.

**25.** Une méthode de production de la protéine ayant la séquence d'acides aminés illustrée à la Figure (3), qui comprend la culture des cellules hôtes de la revendication 23 dans des conditions adéquates, permettant de produire la protéine et de récupérer la protéine ainsi produite.

**26.** Une méthode permettant d'obtenir l'ADN de la revendication 19, qui comprend l'isolation de l'ADN génomique total des oocystes d'Eimeria tenella; la préparation de fragments d'ADN de l'ADN génomique ainsi isolé; la liaison des fragments ainsi préparés dans un vecteur de clonage approprié; la soumission de l'ADN des clones ainsi préparés à l'hybridation avec des oligonucléotides contenant des ou complémentaires aux séquences de l'acide nucléique présent dans la séquence d'acide nucléique illustrée à la Figure (3) pour identifier les clones adéquats; et l'isolation à partir de l'ADN des clones adéquats qui code la protéine et a la séquence d'acide nucléique illustrée à la Figure (3).

Figure 1: Amino Acid Sequence of the 17,000 Dalton Polypeptide Component
of the A4 Antigen

```
        [gln]asp tyr pro thr ala val thr leu asp(cys)lys(glu)ala
             |-------------------PAP------------------------->
                                            |------CH3----------->
                                                            |V7


met asn lys leu arg lys ala ala gly leu pro ala phe glu asp ala val gly
---------------------------CH3------------------------------------------
---------------------------V7-------------------------------------------
     |----------------------------------CN1---------------------------


asp thr phe val leu pro ala tyr(ser his)glu glu ser arg ala ala pro val
--CH3-----| |-------------------CH2'------------------------------------
--------V7----------------->                    |--------V6------------
-----------------------------CN1------------------------------------->
                                                      |----R2---------


ala glu thr leu trp lys thr glu ile cys pro lys val leu gly gly gly arg
---CH2'--->                       |------------------V4-----------------
-V6---| |------------------------------------------V2-----------------
-----------------------------------R2-----------------------------------
                                                      |------CH5-----


ser arg asn val thr glu ala val lys leu thr gly asn phe ala tyr tyr pro
--------V4-----------|  |-------------------V5-------------------------
--------V2-----------|  |-------------------V1-------------------------
--R2--|
-----------------------CH5-------------------------->
|-----------------------------R1-------------------------------------


val thr asp gly lys lys glu cys ser asp ala val glu tyr trp lys gly gly
---------V5----------------|                              |---CH2----
-------------------------V1---------------------------------------------
------------------------R1------------------------------->       |--R4--


leu ser gln phe asn asp thr ile pro pro thr phe gln ala leu asn asp pro
-----------------------------CH2--------------------------------------->
----------V1--------------->
------------------------------------R4----------------------------------
                                                      |---R2'----


val val tyr asn asp arg ala val ser phe val ala leu tyr asn pro lys thr
-------R4------------------->       |----------CH1-------------------
             |----------CH4------------|
-----------------R2'------------------------------------------->


ser pro val val ser(cys)val leu leu gln(cys)pro asn ala gly val gly gly
------------------------------CH1------------------------------------|
```

> indicates peptide sequence may continue, but too weak to follow
| indicates peptide C-terminus
() probable amino acids confirmed by DNA sequence
' indicates secondary sequence
CN: cyanogen bromide fragment; CH: chymotrypsin fragment; R: Arg-C
fragment; V: V8 fragment; PAP: pyroglutamate aminopeptidase treated
17,000 dalton polypeptide.

EP 0 164 176 B1

**Figure 2.**    Restriction Map of the 5.5 kb Insert of Clone 108-1.

17,000 D   8,000 D
peptide    peptide

1000 b.p.

PstI and BalI sites shown are the rightmost sites, but not the only sites.

No sites for BamHI, EcoRV, NruI, SalI, SmaI and PvuI.

Figure 3a

```
                                        AGATCTATCAAGCAATAATCATCTA
CCTCCAAATATATGCTATGAAATGC7AAATT8CGTGAGAGTGATTCGTCACAGCAACGTC
TCATGCAGAGTGCCCGAGAACTGA5GGGAGAAACAGTGGAGTGACCGCGGGTCGCTGGTA
TTTTCTTGCTTTCATTOGCAAACGYGGCATTTTCAAGTGCCATTTTTCTTGTAATCACAT
TAGTTTGCCAGTAAATGAGGGGAATATTCTGGTGTAAGCTGTTCTTCTGGCAGTTTCACG
AGAGTCACACCGTCACCTGGOAGGTAACCTGGAAAGGGGCGGTGGCAGGAATGGCGCAAG
GCATGGAACAATGAAAGCTGAGAGCAGCGTCAAA3GGATGAATTTTCAATTTCACGTTTG
CCCTTAAATCCATTCAAGTGGGCCGAGACCGCTCTCGGAAGYGCAGTCTCGTTTGCGATT
GCATTMCCTGCACACACCTATGACGACGTACGGTGTTGGGCAGAACCTGAACATAGCGTT
TACGTCTAMAGCCGCAGCCCAAAGAAACTCTGCATACTTTTGCCAAGATATTTCAAATAA
AACCTCTTTGCCGAATTGTATTTTCACCCTCTATCTACTATTTCCTGCCCACTATGAGAG
GCAGCAAGC7GTAGCGTGCCTTCCAATGGCCAGCACCAGCGCGCCAG7TAGGGCAGCAGC
TGTCAACCTCGCTGTCATCTGTCAACAGGCCGCCAGAACTCTTCCCATATCTGTCAAAAC
ATATTTATCTGCTCACTTTACAGTTTCTGTACAGTCACTTTTGCATATTATACAATTACT

                         MetAlaArgLeuSerPheValSerLeuLeuSerLeuSer
GTACAGTCATATTTGCTCAAAATGGCTCGTCTTTCTTTTGTTTCTCTTCTTTCTCTGTCA
                                        •
LeuLeuPheGlyGlnGlnAlaValArgAlaGlnAspTyrProThrAlaV<----------
CTGCTCTTCGGGCAGCAAGCAGTCAGAGCTCAGGATTACCCAACAGCAGGTGGGCTTTTC

-------------------Intron A----------------------------------
CGCTAGCTGTTTTTGGTCCGATAGCATCGGAGCATCTCCCAAAACGAGGTGCATTCACC

-------------------------------->alThrLeuAspCysLysGluAlaMetAsn
TTTTGCATGTTGTGTGCGGAAATTTTATCAGTTACGCTGGACTGTAAAGAAGCGATGAAC

LysLeuArgLysAlaAlaGlyLeuProAlaPheGluAspAlaValGlyAspThrPheVal
AAGCTGAGAAAAGCAGCAGGACTTCCTGCATTCGAAGATGCTGTGGGAGACACATTTGTT
                                            •
LeuProAlaTyrSerHisGluGluSerArgAlaAlaProValAlaGluThrLeuTrpLys
CTACCAGCATACTCGCATGAAGAGTCTAGGGCGGCACCAGTAGCTGAAACTCTCTGGAAG

ThrGluIleCysProLysValLeuGly<---------------------------------
ACGGAGATATGCCCCAAAGTCTTAGGAGTAAGCCGTCCACGGCCTTGCATCGTCATGATG
```

| FIG 3 | FIG 3a | FIG 3b | FIG 3c |

Figure 3b

```
-------------------Intron B-------------------------------
TAGTAGGTGTTCTGAGCAGCTTCGTTCTGTGGAACAAGGAACTACACTGTCCTTGAATTT


--------------------->GlyGlyArgSerArgAsnValThrGluAlaValLysLeu
TTAATCTTTTGTTACGTACAGGGCGGAAGGTCCAGGAACGTTACTGAAGCTGTCAAGTTA

ThrGlyAsnPheAlaTyrTyrProValThrAspGlyLysLysGluCysSerAspAlaVal
ACTGGCAATTTTGCCTACTACCCCGTCACAGACGGCAAAAAAGAGTGCAGCGATGCTGTG

GluTyrTrpLysGlyGlyLeuSerGlnPheAsnAspThrIleProProThrPheGlnAla
GAGTACTGGAAAGGCGGACTTTCTCAGTTCAACGACACAATTCCCCCAACGTTCCAAGCG

LeuAsnAspProValValTyrAsnAspArgAlaValSerPheValAlaLeuTyrAsnPro
TTGAACGACCCCGTTGTGTACAATGACAGGGCTGTTTCCTTTGTCGCCCTATACAACCCC

                                                          ••
LysThrSerProValValSerCysValLeuLeuGlnCysProAsnAlaGlyValGlyGly
AAAACCAGCCCCGTTGTCAGTTGCGTGCTCCTCCAGTGCCCTAATGCAGGTGTTGGTGGA

                    ◆
ArgArgLeuAlaAlaGlyThrThrAspAlaValIleCysLeuThrAsnProAlaProLeu
CGCAGGCTTGCGGCAGGCACGACAGACGCTGTCATTTGCTTGACAAATCCGGCTCCTTTG

GluAlaArgSerGlnProPheAs<-----------------------------------
GAAGCAAGGTCACAACCATTCGAGTGAGAGTCAGCTGGTCGCCACTGCAACATGCATCAA

-------------------Intron C-------------------------------
TGCGGCAGGTTACACTGGGGGGTCTTGAGGTTGGTTGAAGCGCAATCTTCTAATACTTGTT

------------------------->pAspGluGlnTrpLysLysIleValAspSerLe
TGTAATGTTTGTAATGTTTGCGTGCAGCGACGAGCAATGGAAGAAAATTGTTGACTCTCT

uSerLeuSerGluGluGluGluGluLysGlyGlyValSerProValValProSerValAl
ATCTCTCTCTGAGGAAGAGGAAGAGAAGGGCGGAGTTTCTCCAGTCGTCCCTTCAGTAGC

                                  ◆◆
aLeuIleSerAlaAlaValIleSerAlaPheAlaLeuPhe
CCTCATCTCTGCGGCGGTCATCTCCGCTTTCGCTCTCTTTTAGGCGGGCGCCGGTTGTTA

GTGACACACCAGCATTGGACAGATATGGCGGCGCAAGTTCCTTCCTGAGTGAAATCCTTG

AGTGACAAACGAGCACCTCTCCTGGACGAAATGTGATGAATTAAGACAGCTTTGGTTGTT

TGAAGTGTATGCAAAAGCTACATTTGTAGGGCCCTTTTATAGGATAATCGGAGGAAGCGC

AATTTTATTTAAAACCCTTGCAGAGAGTCGCCACGTGCGAGTGCAAGTGTTGCGCAGTGT

GTGCTGCCAAATGAAATTCTCGATCTTTAGTGTACTCAAGCCAGAAGTTTCGGCGTTGAT

GTACCCGCCGGTGGTATCTGCCATGCCATGCCTGCCTGTTTGGGCAGTACAACCTCATAC
```

Figure 3 C

CAAGTGGCTTGTGTCATGGCATGTGTGGCCAAGCTACTTTTAGAGGGACAACAATGGGGA

TATTTTGAAGTATTTCGGATAAATACTCATCTGCTGTCCCTACCCACTGAGGCGCCATGG

TGTTACCTTCCTCATTTGAAGGGGAAAACTTGGTTGATAATTTCTTGTCCTTCAACTTGT

CTTGATAAATCGAAGATTATATTGTAGATAGTATACGTGGTGAACAGTTTTTAGGGAAGA

CTGTAAACCACAAGTTAAACGTAGTCGGAATTC


Legend

* Initial amino acid of 17,000 dalton peptide
** Final amino acid of 17,000 dalton peptide
+ Initial amino acid of 8,000 dalton peptide
++ Final amino acid of 8,000 dalton peptide

Key to ambiguous bases

3 = Probably C
5 = Probably A
7 = Maybe C
8 = Maybe T
0 = Maybe G
Y = C or T
M = C or G